# EUROPEAN PATENT APPLICATION

(11) **EP 1 990 425 A1**
(43) Date of publication of application: **12.11.2008**
(21) Application number: 07301027.4
(22) Date of filing: 10.05.2007
(51) Int. Cl.: C12Q 1/68

(54) **Diagnostic of immune graft tolerance**

(71) Applicant: TCLAND R, 44200 Nantes (FR); INSTITUT NATIONAL DE LA SANTE ET DE LA RECHERCHE MEDICALE (INSERM), 75013 Paris (FR)
(72) Inventor: The designation of the inventor has not yet been filed
(74) Representative: Warcoin, Jacques

(57) **Abstract**

The present invention concerns a method for the in vitro diagnosis of a graft tolerant phenotype, comprising : determining from a grafted subject biological sample an expression profile comprising, or consisting of, 8 genes, and optionally at least one among 41 further genes, identified in the present invention as differentially expressed between graft tolerant subjects and subjects in chronic rejection, optionally measuring other parameters, and determining the presence or absence of a graft tolerant phenotype from said expression profile and optional other parameters. Said method may further comprise, if said subject is diagnosed as a graft non-tolerant subject, diagnosing from the expression profile if said subject is developing chronic rejection. The invention further concerns kits and oligonucleotide microarrays suitable to implement said method.

## Description

The present invention concerns a method for the in vitro diagnosis of a graft tolerant or graft non-tolerant phenotype, comprising : determining from a grafted subject biological sample an expression profile comprising eight genes, and optionally at least one gene among 41 further genes identified in the present invention as differentially expressed between graft tolerant subjects and subjects in chronic rejection, optionally measuring other parameters, and determining the presence of a graft tolerant or graft non-tolerant phenotype from said expression profile and optional other parameters. Said method may further comprise, if said subject is diagnosed as a graft non-tolerant subject, diagnosing from the expression profile if said subject is developing chronic rejection. The invention further concerns kits and oligonucleotide microarrays suitable to implement said method. It may further concern protein microarrays as well as other methods of transcriptional and genomic analysis.

Currently, the long-term survival of an allograft is depending on the continuous administration of immunosuppressive drugs. Indeed, an interruption of the immunosuppressive treatment generally leads to an acute or chronic rejection, particularly in case of an early or abrupt diminution.

However, long-term immunosuppressive treatments lead to severe side effects such as chronic nephrotoxicity, an increased susceptibility to opportunistic infections, and a dose-dependant increased propensity to develop virus induced malignancies (1).

Despite the difficulties encountered by many attempts to induce a persistent tolerance to allografts in human, it has been observed that some patients can maintain the tolerance to their graft without any immunosuppressive treatment (ref 2), demonstrating that a state of operational tolerance may naturally occur, even in humans.

In the case of kidney graft, the real proportion of tolerant grafted subjects may be underestimated. Indeed, although the possibility to progressively stop the immunosuppressive treatment has never been investigated, a significant proportion of kidney grafted subject accept their graft with a minimal dose of immunosuppressive drug (cortisone monotherapy, <10mg a day) (2). In addition, among patients developing post-transplantation lymphoproliferative disorders, leading to the interruption of their immunosuppressive treatment, some does not reject their graft.

Thus, a significant proportion of kidney grafted subjects might display an unsuspected, total or partial, immune operational tolerance state to their graft. It would therefore be very useful to have a method to diagnose, without any previous modification of the immunosuppressive treatment, the level of immune tolerance of grafted subjects taken individually. Indeed, this would allow for an ethically acceptable, progressive, total or partial withdrawal of immunosuppressive drugs in subject with a high enough level of graft tolerance. Although well known biological parameters are used by clinicians for the evaluation of renal function (creatinine and urea serum concentrations and clearance), these parameters are not sufficient for a precise diagnosis of tolerance or rejection and most importantly, have no predictive value. Currently, only a biopsy of the grafted kidney allows, through the analysis of the presence or absence of several histological lesion types (3), for the precise evaluation of said grafted kidney functionality. However, a biopsy is an invasive examination, which is not without danger for the grafted organ, and is thus usually not performed on grafted subjects that have stable biological parameters values. In addition, the variability of the diagnosis, due to the subjectivity of the analysis, is a drawback of the histological examination of biopsies. A non-invasive accurate and reliable method of diagnosis of a graft tolerant phenotype is thus needed.

In addition, in the case of many grafted organ, when the values of standard biological parameters allow for the diagnostic of chronic rejection, the rejection process is already in progress and, although it may in certain cases be stopped, the lesions that have been induced generally cannot be reversed. Moreover, to confirm the diagnostic, a biopsy of the grafted organ is usually performed, which is, as stated before, not without danger. It would thus also be very valuable to have a non-invasive method allowing to diagnose chronic rejection at the earlier steps of the rejection process, which would permit to adapt the immunosuppressive treatment and might in some cases prevent the chronic rejection process.

Finally, a non-invasive method for an early and reliable diagnosis of a graft tolerant or non-tolerant phenotype would be very useful in clinical research, since it would allow for relatively short (6 months to 1 year), and thus less expensive, clinical trial studies.

At the present time, few genome-wide studies have been carried out in humans on the modifications of gene expression patterns after kidney transplant. In addition, these studies focused on the identification of genes implicated in graft acute or chronic rejection, and not in graft tolerance. From the comparison of the expression level of about 12000 unique genes in tolerant patients versus patients in chronic rejection, the inventors identified a list of 49 genes that are significantly differentially expressed between the two groups of patients, and that permits a reliable identification of graft-tolerant or graft non-tolerant patients among a group of grafted patients. Among these 49 genes, 8 are particularly pertinent with respect to the tolerance state of the grafted patients, and permit alone or in combination with at least one of the other 41 genes, a reliable identification of graft-tolerant or graft non-tolerant patients among a group of grafted patients.

Thanks to the identification of these genes that are significantly differentially expressed between tolerant patients and patients in chronic rejection, it is now possible to use a non-invasive method of in vitro diagnosis of a graft tolerant or, on the contrary, a graft non-tolerant phenotype. Such a method allows for the identification of grafted subject for whom a progressive, total or partial withdrawal of immunosuppressive drugs is possible. It also permits an early diagnosis of a chronic rejection process in patients whose biological parameters levels are still normal. Moreover, the diagnosis may be performed from a blood sample, which is completely harmless for the tested grafted subject.

The invention thus concerns a method for the in vitro diagnosis of a graft tolerant or non-tolerant phenotype, comprising:
(a) determining from a grafted subject biological sample an expression profile comprising, or consisting of, the 8 genes from Table 1, and
(b) comparing the obtained expression profile with at least one reference expression profile, and
(c) determining the graft tolerant or graft non-tolerant phenotype from said comparison

In one embodiment of the above method according to the invention, the expression profile further comprises at least one of the genes from Table 2. In this case, the expression profile may comprise 1, 2, 3, 4, 5 or more, such as about 10, 15, 20, 25, 30, 35, 40 or even the 41 genes from Table 2. In a particular embodiment, the expression profile comprises or consists of 49 genes (the 8 genes of Table 1 and the 41 genes of Table 2).

According to the present invention, a "graft tolerant phenotype" is defined as a state of tolerance of a subject to his graft. A "state of tolerance" means that this subject (referred to as a "graft tolerant subject") does not reject his graft in the absence of an immunosuppressive treatment with a well functioning graft. In contrast, a "graft non-tolerant phenotype" refers to the absence in said subject of a state of tolerance, meaning that said subject (referred to as a "graft non-tolerant subject") would, at the time of the diagnosis, reject its graft if the immunosuppressive treatment was withdrawn. While the population of graft tolerant subjects only includes subjects in a state of tolerance to their graft, the population of graft non-tolerant subjects thus includes all other subjects and is composed of a variety of different states: patients already suffering from obvious chronic rejection, patients at the early non symptomatic stage of chronic rejection, but also stable patients, which cannot at this time be considered as tolerant but who may later develop a graft tolerant phenotype. Indeed, it must be understood that the mechanisms of tolerance are complex and still not elucidated, and the cellular and molecular processes of tolerance induction may require a prolonged laps of time. Thus, while the population of graft tolerant subjects only includes subjects who have already reached a stable state of tolerance to their graft, the population of graft non-tolerant subjects is heterogeneous and includes all other subjects, i.e. both subjects in the process of developing chronic rejection and subjects in the process of developing tolerance.

Immunosuppressive drugs that may be employed in transplantation procedures include azathioprine, methotrexate, cyclophosphamide, FK-506, rapamycin, corticosteroids, and cyclosporins. These drugs may be used in monotherapy or in combination therapies.

In the case of kidney graft, the following immunosuppressive protocols are usually used.

Subjects with primary kidney graft generally receive an induction treatment consisting of 2 injections of basiliximab (Simulect®, a chimeric murine/human monoclonal anti IL2-Rα antibody commercialized by Novartis), in association with tacrolimus (Prograf^{™}, Fujisawa Pharmaceutical, 0.1 mg/kg/day), mycophenolate mofetil (Cellcept^{™}, Syntex Laboratories, Inc, 2 g/day) and corticoids (1 mg/kg/day), the corticoid treatment being progressively decreased of 10 mg every 5 days until end of treatment, 3 months post transplantation.

Subjects with secondary or tertiary kidney graft, or subjects considered at immunological risk (percentage of anti-T PRA previously peaking above 25% or cold ischemia for more than 36 hours), generally receive a short course of anti-thymocyte globulin (ATG) (7 days), in addition from day 0 with mycophenolate mofetil (Cellcept^{™}, Syntex Laboratories, Inc, 2 g/day), and corticosteroids (1 mg/kg/day), then the steroids are progressively tapered of 10 mg every 5 days until end of treatment and finally stopped around 3 months post transplantation. Tacrolimus (Prograf^{™}, Fujisawa Pharmaceutical) is introduced in a delayed manner (at 6 days) at a dose of 0.1 mg/kg/day.

The present invention possesses two major interests:
- first, it permits to diagnose or prognose (i.e. to identify), among patients under immunosuppressive treatment, those who are tolerant to their graft and who could thus benefit from a progressive partial or total withdrawal of the immunosuppressive treatment while remaining tolerant to their graft. Due to the side effects of immunosuppressive treatments, this achievement is really crucial; and
- second, it further permits more precisely to diagnose or prognose (i.e. to identify), among patients under immunosuppressive treatment who are diagnosed by the method according to the invention as graft non-tolerant (i.e. patients that are not diagnosed as graft tolerant) but who are apparently stable in view of their still normal clinical parameters, those who are already at the early steps of chronic graft rejection. Thus, the invention also permits to detect patients who would need a modified immunosuppressive treatment to prevent chronic rejection at the very beginning of the rejection process. In this case, the early adaptation of the immunosuppressive treatment then favors the prevention of chronic rejection.

A "biological sample" may be any sample that may be taken from a grafted subject, such as a serum sample, a plasma sample, a urine sample, a blood sample, a lymph sample, or a biopsy. Such a sample must allow for the determination of an expression profile comprising or consisting of the 8 genes from Table 1 and optionally at least one gene from Table 2. Preferred biological samples for the determination of an expression profile include samples such as a blood sample, a lymph sample, or a biopsy. Preferably, the biological sample is a blood sample, more preferably a peripheral blood sample comprising peripheral blood mononuclear cells (PBMC). Indeed, such a blood sample may be obtained by a completely harmless blood collection from the grafted patient and thus allows for a non-invasive diagnosis of a graft tolerant or non-tolerant phenotype.

By "expression profile" is meant a group of at least 8 values corresponding to the expression levels of the 8 genes of Table 1, with optionally at least one further value corresponding to the expression level of at least one (and in some embodiments all) gene(s) from Table 2, and optionally with further other values corresponding to the expression levels of other genes. Preferably, the expression profile consists of a maximum of 500, 400, 300, 200, preferably 100, 90, 80, 75, more preferably 70, 65, 60, even more preferably 55, 54, 53, 52, 51, 50, or 49 distinct genes, 8 of which are the 8 genes of Table 1, the remaining genes being preferably selected from the 41 genes of Table 2. In a most preferred embodiment, the expression profile consists of the 8 genes of Table 1, since this expression profile has been demonstrated to be particularly relevant for assessing graft tolerance/non-tolerance. In another preferred embodiment, the expression profile consists of 49 genes made of the 8 genes of Table 1 and the 41 genes of Table 2. However, the addition of a restricted number of other genes (not listed in Table 1 nor Table 2) does not significantly reduce the reliability of the test, provided that the 8 genes of Table 1, and optionally at least one gene of Table 2, are analyzed, which is why expression profiles with a maximum of 500 distinct genes, 8 of which are the 8 genes of Table 1 are included in the scope of the invention.

In addition, although the list of 8 genes of Table 1 has been determined as the best expression profile to assess graft tolerance/non-tolerance, the omission of a restricted number of genes from Table 1, for example the omission of 1 or 2 genes from the list of 8 genes of Table 1, still permits to assess graft tolerance, although with less reliability.

The 8 genes that were determined by the inventors to display significantly different expression levels between kidney graft tolerant subjects as defined above (Tol) and kidney transplanted subjects in chronic rejection (CR) are listed in the following Table 1.

**Table 1. 8 genes differentially expressed between kidney transplanted subjects that are tolerant (Tol) or in chronic rejection (CR).**

| **N°** | **Symbol** | **Name** | **Accession Nb (RefSeq)** | **LLocus ID** | **Synonyms** | **UniGeneID** | **LocChr** |
|---|---|---|---|---|---|---|---|
| 1 | BUB1B | BUB1 budding uninhibited by benzimidazoles 1 homolog beta (yeast) | NM_001211 | 701 | BUB1 beta, BUBR1, Bub1A, MAD3L, SSK1, hBUBR1 | Hs.631699 | 15q15 |
| 2 | CDC2 | cell division cycle 2, G1 to S and G2 to M | NM_001786 .2 NM_03337 9.2 | 983 | CDC28A, CDK1, DKFZp686L 20222, MGC11119 5 | Hs.334562 | 10q21.1 |
| 3 | CHEK1 | CHK1 checkpoint homolog (S. pombe) | NM_001274 .2 | 1111 | CHK1 | Hs.24529 | 11 q24-q24 |
| 4 | MS4A1 | membrane-spanning 4-domains, subfamily A, member 1 | NM_152866 .2 NM_02195 0.3 | 931 | B1, Bp35, CD20, LEU-16, MGC3969, MS4A2, S7 | Hs.438040 | 11q12 |
| 5 | RAB30 | RAB30, member RAS oncogene family | NM_014488 .3 | 27314 | Ras-related protein Rab-30 | Hs.40758 | 11q12-q14 |
| 6 | RHOH | ras homolog gene family, member H | NM_004310 .2 | 399 | ARHH, TTF | Hs.160673 | 4p13 |
| 7 | SYNGR3 | synaptogyrin 3 | NM_004209 .4 | 9143 | MGC:20003 | Hs.435277 | 16p13 |
| 8 | TMTC3 | transmembrane and tetratricopeptide repeat containing 3 | NM_181783 .1 | 160418 | SMILE, DKFZp686C 0968, DKFZp686 M1969, DKFZp686 022167, DKFZp686 02342, FLJ90492, | Hs.331268 | 12q21.3 2 |

The 41 further genes also determined by the inventors as relevant for assessing graft tolerance are displayed in the following Table 2.

**Table 2. 41 further genes differentially expressed between kidney transplanted subjects that are tolerant (Tol) or in chronic rejection (CR).**

| N° | Symbol | Name | AccessionNb (RefSeq) | Locus ID | Synonyms | UniGeneID | LocChr |
|---|---|---|---|---|---|---|---|
| 9 | AFP | Alpha-fetoprotein | NM_001134.1 | 174 | Alpha-fetoglobulin, FETA,HPAFP | Hs.518808 | 4q11-q13 |
| 10 | AKR1C1 | Aldo-keto reductase family 1, member C1 (dihydrodiol dehydrogena se 1; 20-alpha (3-alpha)-hydroxysteroi d dehydrogena se) | NM_001353.5 | 1645 | 2-ALPHA-HSD, 20-ALPHA-HSD, C9, DD1, DDH, DDH1, H-37, HAKRC, MBAB, MGC8954 | Hs.460260 | 10p15-p14 |
| 11 | AREG | Amphiregulin (schwannom a-derived growth factor) | NM_001657.2 | 374 | AR, CRDGF, MGC13647, SDGF | Hs.270833 | 4q13-q21 |
| 12 | BRRN1 | Barren homolog (Drosophila) | NM_015341.3 | 23397 | NCAPH, CAP-H, HCAP-H, BRRN, KIAA0074 | Hs.308045 | 2q11.2 |
| 13 | BTLA | B and T lymphocyte associated | NM_181780.2 | 151888 | BTLA1, CD272, FLJ16065, MGC129743 | Hs.445162 | 3q13.2 |
| 14 | C1S | Complement component 1, s subcompone nt | NM_001734.2 NM_201442.1 | 716 | 0 | Hs.458355 | 12p13 |
| 15 | CCL20 | Chemokine (C-C motif) ligand 20 | NM_004591.1 | 6364 | CKb4, LARC, MIP-3a, MIP3A, SCYA20, ST38, exodus-1 | Hs.75498 | 2q33-q37 |
| 16 | CDH2 | Cadherin 2, type 1, N-cadherin (neuronal) | NM_001792.2 | 1000 | CDHN, CDw325, NCAD, Neural-cadherin | Hs.464829 ; Hs.606106 | 18q11.2 |
| 17 | DEPDC1 | DEP domain containing 1 | NM_017779.3 | 55635 | DEP.8, FLJ20354, SDP35 | Hs.445098 | 1p31.2 |
| 18 | DHRS2 | Dehydrogena se/reductase (SDR family) member 2 | NM_005794.2 NM_182908.3 | 10202 | HEP27 | Hs.272499 | 14q11.2 |
| 19 | ELF3 | E74-like factor 3 (ets domain transcription factor, epithelial-specific) | NM_004433.3 | 1999 | EPR-1, ERT, ESE-1, ESX | Hs.67928 | 1 q32.2 |
| 20 | GAGE2 | G antigen 2 | NM_001472.2 | 2574 | MGC120097, MGC96883, MGC96930, MGC96942 | Hs.460641 | Xp11.23 |
| 21 | HBB | Hemoglobin, beta | NM_000518.4 | 3043 | CD113t-C, HBD, beta-globin | Hs.523443 | 11p15.5 |
| 22 | IGFBP3 | Insulin-like growth factor binding protein 3 | NM_00101339 8.1 NM_000598.4 | 3486 | tcag7.703, BP-53, IBP3 | Hs.450230 | 7p13-p12 |
| 23 | IL13RA2 | Interleukin 13 receptor, alpha 2 | NM_000640.2 | 3598 | CD213A2, IL-13R, IL13BP | Hs.336046 | Xq13.1-q28 |
| 24 | LTB4DH | Leukotriene B4 12-hydroxydehy drogenase | NM_012212.2 | 22949 | RP11-16L21.1, MGC34943 EC 1.3.1.48 EC 1.3.1.74 | Hs.584864 | 9q31.3 |
| 25 | MTHFD2 | Methylenetetr ahydrofolate dehydrogena se (NADP+ dependent)2, methenyltetra hydrofolate cyclohydrolas e | NM_00104040 9.1, NM_006636.3 | 10797 | NMDMC | Hs.469030 | 2p13.1 |
| 26 | NR2F1 | Nuclear receptor subfamily 2, group F, member 1 | NM_005654.4 | 7025 | COUP-TFI, EAR-3, EAR3, ERBAL3, NR2F2, SVP44, TCFCOUP1, TFCOUP1, COUP-TFA, COUP-TF1 | Hs.519445 | 5q14 |
| 27 | PARVG | Parvin, gamma | NM_022141.4 | 64098 | 0 | Hs.565777 | 22q13.2-q13 |
| 28 | PCP4 | Purkinje cell protein 4 | NM_006198 | 5121 | PEP-19 | Hs.80296 | 21q22.2 |
| 29 | PLEKHC 1 | Pleckstrin homology domain containing family C (with FERM domain) member 1 | NM_006832.1 | 10979 | FLJ34213, FLJ44462, KIND2, MIG2, UNC112, mig-2, Kindlin-2 | Hs.652309 Hs.645402 | 14q22.2 |
| 30 | PLXNB1 | Plexin B1 | NM_002673.3 | 5364 | KIAA0407, PLEXIN-B1, PLXN5, SEP | Hs.476209 | 3p21.31 |
| 31 | PODXL | Podocalyxin-like | NM_00101811 1.1 NM_005397.2 | 5420 | Gp200, MGC138240, PCLP | Hs.16426 | 7q32-q33 |
| 32 | PPAP2C | Phosphatidic acid phosphatase type 2C | NM_177543.1 NM_003712.2 NM_177526.1 | 8612 | LPP2, PAP-2c, PAP2-g EC 3.1.3.4 | Hs.465506 | 19p13 |
| 33 | PXDN | peroxidasin homolog (Drosophila) | NM_012293.1 | 7837 | D2S448, D2S448E, KIAA0230, MG50, PRG2, PXN | Hs.332197 | 2p25 |
| 34 | RASGRP 1 | RAS guanyl releasing protein 1 | NM_005739.2 | 10125 | CALDAG-GEFI, CALDAG-GEFII, MGC129998, MGC129999, RASGRP, V, hRasGRP1 | Hs.591127 | 15q15 |
| 35 | RBM9 | RNA binding motif protein 9 | NM_00103169 5.1, NM_014309.1 | 23543 | Fox-2, HRNBP2, RTA, dJ106120.3, fxh | Hs.604232 | 22q13.1 |
| 36 | RGN | Regucalcin (senescence marker protein-30) | NM_152869.2 NM_004683.4 | 9104 | CTD-2522E6.2, RC, SMP30 | Hs.77854 | Xp11.3 |
| 37 | SERPIN A3 | serpin peptidase inhibitor, clade A (alpha-1 antiproteinas e, antitrypsin), member 3 | NM_001085.4 | 12 | AACT, ACT, GlG24, GlG25, MGC88254, alpha1-antichymotrypsi n | Hs.534293, Hs.653605 Hs.644859 | 14q32.1 |
| 38 | SERPIN A5 | serpin peptidase inhibitor, clade A (alpha-1 antiproteinas e, antitrypsin), member 5 | NM_000624.3 | 5104 | PAI3, PCI, PLANH3, PROCI | Hs.510334 | 14q32.1 |
| 39 | SLC29A1 | Solute carrier family 29A1 (nucleoside transporters) | NM_00107817 7.1 NM_004955.1 | 2030 | ENT1, MGC1465, MGC3778 | Hs.25450 | 6p21.1-p21.2 |
| 40 | SOX3 | SRY (sex determining region Y)-box 3 | NM_005634.2 | 6658 | MRGH, SOXB | Hs.157429 | Xq27.1 |
| 41 | SPON1 | Spondin 1, extracellular matrix protein | NM_006108.2 | 10418 | KIAA0762, MGC10724, f-spondin, VSGP | Hs.643864 | 11p15.2 |
| 42 | STK6 | Serine/threon ine kinase 6 | NM_198433.1 NM_198437.1 NM_003600.2 NM_ 198434.1 NM_ 198435.1 NM_ 198436.1 | 6790 | AURKA, AIK, ARK1, AURA, AURORA2, BTAK, MGC34538, STK15, STK7, Aurora-A, EC2.7.11.1 | Hs.250822 | 20q13.2-q13.3 |
| 43 | TACC2 | Transforming, acidic coiled-coil containing protein 2 | NM_206862.1 NM_006997.2 NM_206860.1 NM_206861.1 | 10579 | AZU-1, ECTACC | Hs.501252, Hs.643068 | 10q26 |
| 44 | TBX3 | T-box 3 (ulnar mammary syndrome) | NM_016569.3 NM_0059963 | 6926 | TBX3-ISO, UMS, XHL | Hs.129895 | 12q24.1 |
| 45 | TK1 | Thymidine kinase 1, soluble | NM_003258.1 | 7083 | TK2, EC2.7.1.21 | Hs.515122 | 17q23.2-q25.3 |
| 46 | TLE4 | Transducin-like enhancer of split 4 (E(sp1) homolog, Drosophila) | NM_007005.3 | 7091 | KlAA1261, BCE-1, E(spl), ESG, ESG4, GRG4 | Hs.444213 | 9q21.31 |
| 47 | AKR1C2 | aldo-keto reductase family 1, member C2 | NM_001354.4, NM_205845.1 | 1646 | AKR1 C-pseudo, BABP, DD, DD2, DDH2, HAKRD, HBAB, MCDR2, 3-alpha-HSD3, HAKRD | Hs.460260, Hs.567256 | 10p15-p14 |
| 48 49 | SP5 zwilch | Sp5 transcription factor kinetochore associated homolog (Drosophila) | NM_00100384 5.1 NM_017975.3 | 389058 55055 | FLJ10036 FLJ16343, KNTC1AP, MGC111034, hZwilch | Hs.368802 Hs.21331 | 2q31.1 15q22.31 |

The determination of the presence of a graft tolerant or graft non-tolerant phenotype is carried out thanks to the comparison of the obtained expression profile with at least one reference expression profile in step (b).

A "reference expression profile" is a predetermined expression profile, obtained from a biological sample from a subject with a known particular graft state. In particular embodiments, the reference expression profile used for comparison with the test sample in step (b) may have been obtained from a biological sample from a graft tolerant subject ("tolerant reference expression profile"), and/or from a biological sample from a graft non-tolerant subject ("non-tolerant reference expression profile"). Preferably, a non-tolerant expression profile is an expression profile of a subject suffering from chronic rejection.

Preferably, at least one reference expression profile is a tolerant reference expression profile. Alternatively, at least one reference expression profile may be a non-tolerant reference expression profile. More preferably, the determination of the presence or absence of a graft tolerant phenotype is carried out by comparison with at least one tolerant and at least one non-tolerant (preferably chronic rejection) reference expression profiles. The diagnosis (or prognostic) may thus be performed using one tolerant reference expression profile and one non-tolerant (preferably chronic rejection) reference expression profile. Advantageously, to get a stronger diagnosis, said diagnosis is carried out using several tolerant reference expression profiles and several non-tolerant reference expression profiles.

The comparison of a tested subject expression profile with said reference expression profiles can be done using the PLS regression (Partial Least Square) which aim is to extract components, which are linear combinations of the explanatory variables (the genes), in order to model the variable response (eg: 0 if CR, 1 if TOL). The PLS regression is particularly relevant to give prediction in the case of small reference samples. The comparison may also be performed using PAM (predictive analysis of microarrays) statistical method. A non supervised PAM 3 classes statistical analysis is thus performed. Briefly, tolerant reference expression profiles, non-tolerant (preferably chronic rejection) reference expression profiles, and the expression profile of the tested subject are subjected to a clustering analysis using non supervised PAM 3 classes statistical analysis. Based on this clustering, a cross validation (CV) probability may be calculated (CVₜₒₗ), which represents the probability that the tested subject is tolerant. In the same manner, another cross validation probability may be calculated (CVₙₒₙ₋ₜₒₗ), which represents the probability that the tested subject is non-tolerant. The diagnosis is then performed based on the CVₜₒₗ and/or CVₙₒₙ₋ₜₒₗ probabilities. Preferably, a subject is diagnosed as a tolerant subject if the CVₜₒₗ probability is of at least 0.5, at least 0.6, at least 0.7, at least 0.75, at least 0.80, at least 0.85, more preferably at least 0.90, at least 0.95, at least 0.97, at least 0.98, at least 0.99, or even 1.00, and the CVₙₒₙ₋ₜₒₗ probability is of at most 0.5, at most 0.4, at most 0.3, at most 0.25, at most 0.20, at most 0.15, at most 0.10, at most 0.05, at most 0.03, at most 0.02, at most 0.01, or even 0.00. Otherwise, said subject is diagnosed as a graft non-tolerant subject.

In addition, the method according to the invention further permits to diagnose if a graft non-tolerant subject is already in the process of developing a chronic graft rejection. Indeed, when chronic rejection reference expression profiles are used, the CVₙₒₙ₋ₜₒₗ probability is then a CV_{CR} probability, i.e. the probability that the tested subject is undergoing chronic rejection. Then, a more precise diagnosis of this graft non-tolerant subject may be performed based on the CVₜₒₗ and CV_{CR} probabilities. Preferably, a graft non-tolerant subject is diagnosed as developing a chronic rejection if the CV_{CR} probability is of at least 0.5, at least 0.6, at least 0.7, at least 0.75, at least 0.80, at least 0.85, more preferably at least 0.90, at least 0.95, at least 0.97, at least 0.98, at least 0.99, or even 1.00, and the CVₜₒₗ probability is of at most 0.5, at most 0.4, at most 0.3, at most 0.25, at most 0.20, at most 0.15, at most 0.10, at most 0.05, at most 0.03, at most 0.02, at most 0.01, or even 0.00.

Thus, in an embodiment of any method according to the invention, said method further comprises, if said subject is diagnosed as a graft non-tolerant subject, diagnosing from the expression profile if said subject is developing chronic rejection.

The expression profile may be determined by any technology known by a man skilled in the art. In particular, each gene expression level may be measured at the genomic and/or nucleic and/or proteic level. In a preferred embodiment, the expression profile is determined by measuring the amount of nucleic acid transcripts of each gene. In another embodiment, the expression profile is determined by measuring the amount of each gene corresponding protein.

The amount of nucleic acid transcripts can be measured by any technology known by a man skilled in the art. In particular, the measure may be carried out directly on an extracted messenger RNA (mRNA) sample, or on retrotranscribed complementary DNA (cDNA) prepared from extracted mRNA by technologies well-know in the art. From the mRNA or cDNA sample, the amount of nucleic acid transcripts may be measured using any technology known by a man skilled in the art, including nucleic microarrays, quantitative PCR, and hybridization with a labelled probe.

In a preferred embodiment, the expression profile is determined using quantitative PCR. Quantitative, or real-time, PCR is a well known and easily available technology for those skilled in the art and does not need a precise description.

In a particular embodiment, which should not be considered as limiting the scope of the invention, the determination of the expression profile using quantitative PCR may be performed as follows. Briefly, the real-time PCR reactions are carried out using the TaqMan Universal PCR Master Mix (Applied Biosystems). 6 µl cDNA is added to a 9 µl PCR mixture containing 7.5 µl TaqMan Universal PCR Master Mix, 0.75 µl of a 20X mixture of probe and primers and 0.75µl water. The reaction consisted of one initiating step of 2 min at 50 deg. C, followed by 10 min at 95 deg. C, and 40 cycles of amplification including 15 sec at 95 deg. C and 1 min at 60 deg. C. The reaction and data acquisition can be performed using the ABI PRISM 7900 Sequence Detection System (Applied Biosystems). The number of template transcript molecules in a sample is determined by recording the amplification cycle in the exponential phase (cycle threshold or C_{T}), at which time the fluorescence signal can be detected above background fluorescence. Thus, the starting number of template transcript molecules is inversely related to C_{T}.

In another preferred embodiment, the expression profile is determined by the use of a nucleic microarray.

According to the invention, a "nucleic microarray" consists of different nucleic acid probes that are attached to a substrate, which can be a microchip, a glass slide or a microsphere-sized bead. A microchip may be constituted of polymers, plastics, resins, polysaccharides, silica or silica-based materials, carbon, metals, inorganic glasses, or nitrocellulose. Probes can be nucleic acids such as cDNAs ("cDNA microarray") or oligonucleotides ("oligonucleotide microarray"), and the oligonucleotides may be about 25 to about 60 base pairs or less in length.

To determine the expression profile of a target nucleic sample, said sample is labelled, contacted with the microarray in hybridization conditions, leading to the formation of complexes between target nucleic acids that are complementary to probe sequences attached to the microarray surface. The presence of labelled hybridized complexes is then detected. Many variants of the microarray hybridization technology are available to the man skilled in the art, such as those described in patents or patent applications US 5,143,854 (4); US 5,288,644 (5); US 5,324,633 (6); US 5,432,049 (7); US 5,470,710 (8); US 5,492,806 (9); US 5,503,980 (10); US 5,510,270 (11); US 5,525,464 (12); US 5,547,839 (13); US 5,580,732 (14); US 5,661,028 (15); US 5,800,992 (16); WO 95/21265 (17); WO 96/31622 (18); WO 97/10365 (19); WO 97/27317 (20); EP 373 203 (21); and EP 785 280 (r22); the disclosures of which are herein incorporated by reference.

In a preferred embodiment, the nucleic microarray is an oligonucleotide microarray comprising, or consisting of, 8 oligonucleotides specific for the 8 genes from Table 1, and optionally at least one (and in some cases all) gene(s) from Table 2. Preferably, the oligonucleotides are about 50 bases in length.

Suitable microarray oligonucleotides specific for any gene from Table 1 or Table 2 may be designed, based on the genomic sequence of each gene (see Table 1 Genbank accession numbers), using any method of microarray oligonucleotide design known in the art. In particular, any available software developed for the design of microarray oligonucleotides may be used, such as, for instance, the OligoArray software (available at http://berry.engin.umich.edu/oligoarray/), the GoArrays software (available at http://www.isima.fr/bioinfo/goarrays/), the Array Designer software (available at http://www.premierbiosoft.com/dnamicroarray/index.html), the Primer3 software (available at http://frodo.wi.mit.edu/primer3/primer3_code.html), or the Promide software (available at http://oligos.molgen.mpg.de/).

In a particular embodiment of the above method according to the invention, the expression profile further comprises at least one of the genes from Table 3. In this case, the expression profile may comprise 1, 2, 3, 4, 5, 6, 7 or more, such as about 10, 15, 20, 25, 30 or even 40, 50, 60, 70, 80 or even the 102 genes from Table 3.

The additional gene(s) of Table 3 may be analyzed either simultaneously in the same expression profile as the 8 genes from Table 1, and optionally in the same expression profile as at least one gene of Table 2, or as a distinct expression profile. More precisely, the determination of the expression levels of the additional gene(s) of Table 3 may be determined in a common same experiment as those of Table 1, and optionally of Table 2, or in a separate experiment. In addition, the analysis of the results, in particular the comparison with at least one reference expression profile, may be done either in a single common expression profile comprising both genes of Table 1 and Table 3, and optionally Table 2, or as two distinct expression profiles comprising respectively 1) genes of Table 1, and optionally Table 2, and 2) at least one gene from Table 3 (for instance the 102 genes from Table 3).

In a particular embodiment of the second case, the method according to the invention as described above further comprises between steps (b) and (c) the steps of:
(b1) obtaining from a grafted subject biological sample an expression profile comprising, or consisting of, at least one gene (for instance 1, 2, 3, 4, 5, 6, 7 or more, such as about 10, 15, 20, 25, 30 or even 40, 50, 60, 70, 80 or even the 102 genes) from Table 3,
(b2) comparing the obtained expression profile with at least one reference expression profile,
wherein in step (c), the graft tolerant or graft non-tolerant phenotype is determined from the comparison of both step (b1) and step (b2).

Indeed, the genes displayed in following Table 3 are further genes determined by the inventors as being relevant for the appreciation of the operational tolerance state of kidney grafted patients, and may thus be used in addition to the genes of Tables 1 and 2 identified here.

**Table 3. 102 genes differentially expressed between kidney transplanted subjects that are tolerant (Tol) or in chronic rejection (CR).**

| **N°** | **Symbol** | **Name** | **Accession Nb** | **LLocus ID** | **Synonyms** | **RefSeq** | **UniGene ID** | **LocChr** |
|---|---|---|---|---|---|---|---|---|
| 1 | ADAMTS7 | a disintegrin-like and metalloprotease (reprolysin type) with thrombospondin type 1 motif, 7 | NM_014272 | 11173 | ADAM-TS7, DKFZp434H 204 | NM_014272 | Hs.16441 | 15q24.2 |
| 2 | ANPEP | alanyl (membrane) aminopeptidase (aminopeptidase N, aminopeptidase M, microsomal aminopeptidase, CD13, p150) | NM_001150 | 290 | CD13, LAP1, PEPN, gp150 | NM_001150 | Hs.1239 | 15q25-q26 |
| 3 | ANXA2 | annexin A2 | NM_004039 | 302 | ANX2, LIP2, LPC2, CAL1H, LPC2D, ANX2L4 | NM_004039 | Hs.46286 4 | 15q21-q22 |
| 4 | ANXA4 | annexin A4 | NM_001153 | 307 | ANX4 | NM_001153 | Hs.42298 6 | 2p13 |
| 5 | ARPC3B | actin related protein 2/3 complex, subunit 3B, 21kDa | AL133174 | 87171 | dJ470L14.3 | NG_002363 | 0 | 20q13.1 3 |
| 6 | BDP1 | B double prime 1, subunit of RNA polymerase III transcription initiation factor IIIB | NM_018429 | 55814 | TFC5, TFNR, TAF3B1, KIAA1241, KIAA1689, TFIIIB90, HSA238520 , TFIIIB150 | NM_018429 | Hs.27280 8 | 5q12-q13 |
| 7 | BLK | B lymphoid tyrosine kinase | NM_001715 | 640 | MGC10442 | NM_001715 | Hs.38990 0 | 8p23-p22 |
| 8 | BUB1 | BUB1 budding uninhibited by benzimidazoles 1 homolog (yeast) | NM_004336 | 699 | 0 | NM_004336 | Hs.28747 2 | 2q14 |
| 9 | C3AR1 | complement component 3a receptor 1 | NM_004054 | 719 | AZ3B, C3AR, HNFAG09 | NM_004054 | Hs.15593 5 | 12p13.3 1 |
| 10 | C5orf13 | chromosome 5 open reading frame 13 | NM_004772 | 9315 | P311, PTZ17, D4S114, PRO1873 | NM_004772 | Hs.50874 1 | 5q22.2 |
| 11 | CCR6 | chemokine (C-C motif) receptor 6 | NM_031409 | 1235 | BN-1, CKR6, DCR2, CKRL3, DRY-6, GPR29, CKR-L3, CMKBR6, GPRCY4, STRL22, GPR-CY4 | NM_004367 | Hs.46468 | 6q27 |
| 12 | CD33 | CD33 antigen (gp67) | NM_001772 | 945 | p67, SIGLEC-3 | NM_001772 | Hs.83731 | 19q13.3 |
| 13 | CD7 | CD7 antigen (p41) | NM_006137 | 924 | GP40, TP41, Tp40, LEU-9 | NM_006137 | Hs.36972 | 17q25.2 -q25.3 |
| 14 | CENPE | centromere protein E, 312kDa | NM_001813 | 1062 | KIF10 | NM_001813 | Hs.75573 | 4q24-q25 |
| 15 | L26953 | chromosomal protein mRNA, complete cds. | L26953 | 0 | 0 | 0 | 0 | 0 |
| 16 | CLEC2 | C-type lectin-like receptor-2 | NM_016509 | 51266 | 0 | NM_016509 | Hs.40979 4 | 12p13.3 1 |
| 17 | E2F5 | E2F transcription factor 5, p130-binding | NM_001951 | 1875 | E2F-5 | NM_001951 | Hs.44790 5 | 8q21.2 |
| 18 | F2 | coagulation factor II (thrombin) | NM_000506 | 2147 | PT | NM_000506 | Hs.76530 | 11p11-q12 |
| 19 | FKBP1A | FK506 binding protein 1A, 12kDa | M80199 | 2280 | FKBP1, PKC12, PKCI2, FKBP12, PPIASE, FKBP-12, FKBP12C | NM_000801 | Hs.37463 8 | 20p13 |
| 20 | FKRP | fukutin related protein | NM_024301 | 79147 | MDC1C, LGMD21, MGC2991, FLJ12576 | 0 | Hs.19326 1 | 19q13.3 3 |
| 21 | FLJ22222 | hypothetical protein FLJ22222 | NM_175902 | 79701 | 0 | NM_024648 | Hs.43623 7 | 17q25.3 |
| 22 | FLJ22662 | hypothetical protein FLJ22662 | BC000909 | 79887 | 0 | NM_024829 | Hs.17847 0 | 12p13.2 |
| 23 | FLRT1 | fibronectin leucine rich transmembrane protein 1 | NM_013280 | 23769 | 0 | NM_013280 | Hs.52375 5 | 11q12-q13 |
| 24 | FOX01A | forkhead box O1A (rhabdomyosarco ma) | NM_002015 | 2308 | FKH1, FKHR, FOXO1 | NM_002015 | Hs.17013 3 | 13q14.1 |
| 25 | FRAG1 | FGF receptor activating protein 1 | AF159621 | 27315 | 0 | NM_014489 | Hs.13396 8 | 11p15.5 |
| 26 | FXYD3 | FXYD domain containing ion transport regulator 3 | X93036 | 5349 | MAT8, PLML, MAT-8 | NM_005971 | Hs.30135 0 | 19q13.1 3 |
| 27 | GCKR | glucokinase (hexokinase 4) regulatory protein | NM_001486 | 2646 | GKRP | NM_001486 | Hs.89771 | 2p23 |
| 28 | GDAP1 | gangliosideinduc ed differentiationass ociated protein 1 | NM_018972 | 54332 | CMT2G, CMT2H, CMT2K, CMT4A | NM_018972 | Hs.16895 0 | 8q13.3 |
| 29 | GDI1 | GDP dissociation inhibitor 1 | NM_001493 | 2664 | GDIL, MRX41, MRX48, OPHN2, XAP-4, RHOGDI, RABGD1A, RABGDIA | NM_001493 | Hs.74576 | Xq28 |
| 30 | GLRX | glutaredoxin (thioltransferase) | AF069668 | 2745 | GRX | NM_002064 | Hs.28988 | 5q14 |
| 31 | GPR32 | G protein-coupled receptor 32 | NM_001506 | 2854 | 0 | NM_001506 | Hs.24812 5 | 19q13.3 |
| 32 | GPX3 | glutathione peroxidase 3 (plasma) | NM_002084 | 2878 | 0 | NM_002084 | Hs.38679 3 | 5q23 |
| 33 | GRSP1 | GRP1-binding protein GRSP1 | XM_114303 | 23150 | KIAA1013 | XM_114303 | Hs.15886 7 | 3p14.2 |
| 34 | HLA-DOB | major histocompatibility complex, class II, DO beta | NM_002120 | 3112 | 0 | NM_002120 | Hs.1802 | 6p21.3 |
| 35 | HMGB2 | high-mobility group box 2 | NM_002129 | 3148 | HMG2 | NM_002129 | Hs.43495 3 | 4q31 |
| 36 | HNRPA1 | heterogeneous nuclear ribonucleoprotein A1 | NM_002136 /NM_03115 7 | 3178 | HNRNPA1 | NM_002136 | Hs.35672 1 | 12q13.1 |
| 37 | HOXA1 | homeo box A1 | NM_005522 | 3198 | HOX1 F, MGC45232 | NM_005522 | Hs.67397 | 7p15.3 |
| 38 | HSPA6 | heat shock 70kDa protein 6 (HSP70B') | NM_002155 | 3310 | 0 | NM_002155 | Hs.3268 | 1q23 |
| 39 | IBSP | integrin-binding sialoprotein (bone sialoprotein, bone sialoprotein II) | NM_004967 | 3381 | BSP, BNSP, SP-II, BSP-II | NM_004967 | Hs.49215 | 4q21-q25 |
| 40 | ILK | integrin-linked kinase | NM_004517 | 3611 | P59 | NM_004517 | Hs.6196 | 11p15.5 -p15.4 |
| 41 | ILT7 | leukocyte immunoglobulin-like receptor, subfamily A (without TM domain), member 4 | NM_012276 | 23547 | LILRA4 | NM_012276 | Hs.40670 8 | 19q13.4 |
| 42 | BC017857 | cDNA clone IMAGE:4690793, with apparent retainedintron. | BC017857 | 0 | 0 | 0 | 0 | 0 |
| 43 | JAK2 | Janus kinase 2 (a protein tyrosine kinase) | NM_004972 | 3717 | 0 | NM_004972 | Hs.43437 4 | 9p24 |
| 44 | KIR2DL2 | killer cell immunoglobulin-like receptor, two domains, long cytoplasmic tail, 2 | NM_014219 | 3803 | CL-43, NKAT6, p58.2, CD158B1 | NM_014219 | Hs.27845 7 | 19q13.4 |
| 45 | KIR2DL4 | killer cell immunoglobulin-like receptor, two domains, long cytoplasmic tail, 4 | NM_002255 | 3805 | 103AS, 15.212, CD158D, KIR103, KIR103AS | NM_002255 | Hs.16608 5 | 19q13.4 |
| 46 | LAK | lymphocyte alpha-kinase | NM_025144 | 80216 | FLJ22670, KIAA1527 | NM_025144 | Hs.51275 3 | 4q26 |
| 47 | LAMC2 | laminin, gamma 2 | NM_005562 | 3918 | EBR2, BM600, EBR2A, LAMB2T, LAMNB2, KALININ | NM_005562 | Hs.54451 | Xq24 |
| 48 | LNPEP | leucyl/cystinyl aminopeptidase | NM_005575 | 4012 | CAP, IRAP, PLAP | NM_005575 | Hs.43882 7 | 5q15 |
| 49 | LST1 | leukocyte specific transcript 1 | AF129756 | 7940 | B144, LST-1, D6S49E | NM_007161 | Hs.43606 6 | 6p21.3 |
| 50 | LTBP3 | latent transforming growth factor beta binding protein 3 | AF011407 | 4054 | LTBP2, DKFZP586 M2123 | NM_021070 | Hs.28901 9 | 11q12 |
| 51 | MARCO | macrophage receptor with collagenous structure | AF035819 | 8685 | SCARA2 | NM_006770 | Hs.67726 | 2q12-q13 |
| 52 | MMP24 | matrix metalloproteinas e 24 (membrane-inserted) | NM_006690 | 10893 | MMP25, MT5-MMP | NM_006690 | Hs.21258 1 | 20q11.2 |
| 53 | MS4A6A | membrane-spanning 4-domains, subfamily A, member 6A | NM_022349 | 64231 | CDA01, MS4A6, 4SPAN3, CD20L3, 4SPAN3.2, MGC22650 | NM_022349 | Hs.37161 2 | 11q12.1 |
| 54 | MYL9 | myosin, light polypeptide 9, regulatory | J02854 | 10398 | LC20, MLC2, MRLC1, MYRL2, MGC3505 | NM_006097 | Hs.43381 4 | 20q11.2 3 |
| 55 | MYL9 | myosin, light polypeptide 9, regulatory | BC002648 | 10398 | LC20, MLC2, MRLC1, MYRL2, MGC3505 | NM_006097 | Hs.43381 4 | 20q11.2 3 |
| 56 | MYST4 | MYST histone acetyltransferase (monocytic leukemia) 4 | NM_012330 | 23522 | qkf, MORF, MOZ2, KIAA0383, querkopf | NM_012330 | Hs.27590 | 10q22.2 |
| 57 | NCF1 | neutrophil cytosolic factor 1 (47kDa, chronic granulomatous disease, autosomal 1) | AF330627 | 4687 | NOXO2, p47phox | NM_000265 | Hs.1583 | 7q11.23 |
| 58 | NFATC2 | nuclear factor of activated T-cells, cytoplasmic, calcineurin-dependent 2 | NM_012340 | 4773 | NFAT1, NFATP | NM_012340 | Hs.35632 1 | 20q13.2 -q13.3 |
| 59 | NOTCH2 | Notch homolog 2 (Drosophila) | NM_024408 | 4853 | hN2 | NM_024408 | Hs.8121 | 1 p13-p11 |
| 60 | NPC2 | Niemann-Pick disease, type C2 | BC002532 | 10577 | HE1, NP-C2, MGC1333 | NM_006432 | Hs.43322 2 | 14q24.3 |
| 61 | OSM | oncostatin M | NM_020530 | 5008 | MGC20461 | NM_020530 | Hs.24815 6 | 22q12.2 |
| 62 | PGRMC1 | progesterone receptor membrane component 1 | NM_006667 | 10857 | MPR, HPR6.6 | NM_006667 | Hs.90061 | Xq22-q24 |
| 63 | PIP5K2B | phosphatidylinosi tol4phosphate 5kinase, type II, beta | NM_003559 | 8396 | Pip4k2B, PIP5KIIB | NM_003559 | Hs.29107 0 | 17q21.2 |
| 64 | PLCB3 | phospholipase C, beta 3 (phosphatidylinos itol-specific) | NM_000932 | 5331 | 0 | NM_000932 | Hs.43713 7 | 11q13 |
| 65 | PLEKHA3 | pleckstrin homology domain containing, family A (phosphoinositide binding specific) member 3 | AF286162 | 65977 | FAPP1, FLJ20067 | NM_019091 | Hs.41086 | 2q31.3 |
| 66 | PPP1R15A | protein phosphatase 1, regulatory (inhibitor) subunit 15A | NM_014330 | 23645 | GADD34 | NM_014330 | Hs.76556 | 19q13.2 |
| 67 | PRCP | prolylcarboxypept idase (angiotensinase C) | NM_005040 | 5547 | PCP, HUMPCP | NM_005040 | Hs.31408 9 | 11q14 |
| 68 | PSME3 | proteasome (prosome, macropain) activator subunit 3 (PA28 gamma; Ki) | NM_176863 | 10197 | Ki, PA28G, REG-GAMMA, PA28-gamma | NM_005789 | Hs.15297 8 | 17q21 |
| 69 | PTGDS | prostaglandin D2 synthase 21 kDa (brain) | M61900 | 5730 | PDS, PGD2, PGDS, PGDS2 | NM_000954 | Hs.44642 9 | 9q34.2-q34.3 |
| 70 | RAD52B | RAD52 homolog B (S. cerevisiae) | BC038301 | 201299 | MGC33977 | NM_145654 | Hs.19441 1 | 17q11.2 |
| 71 | RET | ret proto-oncogene (multiple endocrine neoplasia and medullary thyroid carcinoma 1, Hirschsprung disease) | NM_020975 | 5979 | PTC, MTC1, HSCR1, MEN2A, MEN2B, RET51, CDHF12 | NM_000323 | Hs.35032 1 | 10q11.2 |
| 72 | RGL | RalGDS-like gene | NM_015149 | 23179 | KIAA0959 | NM_015149 | Hs.79219 | 1 q25.2 |
| 73 | RTN2 | reticulon 2 | NM_005619 | 6253 | NSP2, NSPL1 | NM_005619 | Hs.47517 | 19q13.3 2 |
| 74 | SDHB | succinate dehydrogenase complex, subunit B, iron sulfur (Ip) | NM_003000 | 6390 | IP, SDH, SDH1, SDHIP | NM_003000 | Hs.64 | 1p36.1-p35 |
| 75 | SELP | selectin P (granule membrane protein 140kDa, antigen CD62) | NM_003005 | 6403 | CD62, GRMP, PSEL, CD62P, GMP140, PADGEM | NM_003005 | Hs.73800 | 1 q22-q25 |
| 76 | XM_10624 6 | similar to Heat shock protein HSP 90-alpha (HSP 86)(LOC152918), mRNA. | XM_106246 | 0 | 0 | 0 | 0 | 0 |
| 77 | AY032883 | similar to annexin II receptor | AY032883 | 0 | 0 | 0 | 0 | 0 |
| 78 | XM_09390 2 | similar to Immunoglobulin-binding protein 1(CD79a-binding protein 1) (B cell signal transduction moleculealpha 4) (Alpha 4 protein) (LOC166496), mRNA. | XM_093902 | 0 | 0 | 0 | 0 | 0 |
| 79 | XM_16694 1 | similar to Mitochondrial import receptor subunit TOM20homolog (Mitochondrial 20 kDa outer membrane protein) (Outermitochondr ial membrane receptor Tom20) (LOC220368), mRNA. | XM_166941 | 0 | 0 | 0 | 0 | 0 |
| 80 | XM_09277 2 | similar to dJ760C5.1 (exon similar toABCC7(ATP-binding cassette, sub-family C (CFTR/MRP),me mber 7))(LOC164389), mRNA. | XM_092772 | 0 | 0 | 0 | 0 | 0 |
| 81 | XM_16714 6 | similar to EPIDIDYMAL SECRETORY GLUTATHIONE PEROXIDASEP RECURSOR (EPIDIDYMIS-SPECIFIC GLUTATHIONE PEROXIDASE-LIKE PROTEIN)(EGLP ) (LOC221579), mRNA. | XM_167146 | 0 | 0 | 0 | 0 | 0 |
| 82 | SIRT1 | sirtuin (silent mating type information regulation 2 homolog) 1 (S. cerevisiae) | NM_012238 | 23411 | SIR2L1 | NM_012238 | Hs.31176 | 10q22.1 |
| 83 | SLC29A2 | solute carrier family 29 (nucleoside transporters), member 2 | NM_001532 | 3177 | ENT2, DER12, HNP36 | NM_001532 | Hs.32951 | 11q13 |
| 84 | SMS | spermine synthase | AD001528 | 6611 | SpS, SPMSY | NM_004595 | Hs.44903 2 | Xp22.1 |
| 85 | SPTLC2 | serine palmitoyltransfer ase, long chain base subunit 2 | AF111168 | 9517 | LCB2, SPT2, KIAA0526 | 0 | Hs.59403 | 14q24.3 -q31 |
| 86 | ST13 | suppression of tumorigenicity 13 (colon carcinoma) (Hsp70 interacting protein) | BC015317 | 6767 | HIP, HOP, P48, SNC6, HSPABP, FAM10A1, HSPABP1, PR00786 | NM_003932 | Hs.37719 9 | 22q13.2 |
| 87 | STIM1 | stromal interaction molecule 1 | NM_003156 | 6786 | GOK, D11S4896E | NM_003156 | Hs.74597 | 11p15.5 |
| 88 | STRBP | spermatid perinuclear RNA binding protein | NM_018387 | 55342 | SPNR, MGC3405, FLJ11307, FLJ14223, FLJ14984, MGC21529, DKFZp434N 214 | NM_018387 | Hs.28765 9 | 9q33.3 |
| 89 | SULT1B1 | sulfotransferase family, cytosolic, 1B, member 1 | NM_014465 | 27284 | ST1 B2, SULT1B2, MGC13356 | NM_014465 | Hs.12974 2 | 4q13.3 |
| 90 | TAF1 C | TATA box binding protein (TBP)-associated factor, RNA polymerase I, C, 110kDa | NM_005679 | 9013 | SL1, TAFI95, TAFI110, MGC:39976 | NM_005679 | Hs.15302 2 | 16q24 |
| 91 | TALDO1 | transaldolase 1 | AF058913 | 6888 | TAL, TAL-H, TALDOR | NM_006755 | Hs.43867 8 | 11p15.5 -p15.4 |
| 92 | TCTEL1 | t-complex-associated-testis-expressed 1-like 1 | NM_006519 | 6993 | CW-1, tctex-1 | NM_006519 | Hs.26694 0 | 6q25.2-q25.3 |
| 93 | TERA | TERA protein | NM_021238 | 58516 | 0 | NM_021238 | Hs.35622 3 | 12p11 |
| 94 | TIMM17A | translocase of inner mitochondrial membrane 17 homolog A (yeast) | AF106622 | 10440 | TIM17, TIM17A | NM_006335 | Hs.20716 | 1 q32.1 |
| 95 | TLN1 | talin 1 | NM_006289 | 7094 | TLN, KIAA1027 | NM_006289 | Hs.37500 1 | 9p13 |
| 96 | TPM1 | tropomyosin 1 (alpha) | NM_000366 | 7168 | CMH3, TMSA | NM_000366 | Hs.13389 2 | 15q22.1 |
| 97 | TRAF5 | TNF receptorassociat ed factor 5 | U69108 | 7188 | RNF84, MGC:39780 | NM_004619 | Hs.38568 5 | 1q32 |
| 98 | UHRF1 | ubiquitin-like, containing PHD and RING finger domains, 1 | NM_013282 | 29128 | Np95, ICBP90, RNF106, FLJ21925 | NM_013282 | Hs.10810 6 | 19p13.3 |
| 99 | WNT16 | wingless-type MMTV integration site family, member 16 | NM_016087 | 51384 | 0 | NM_016087 | Hs.27237 5 | 7q31 |
| 100 | YPEL2 | yippee-like 2 (Drosophila) | XM_371070 | 388403 | FKSG4 | XM_371070 | Hs.36867 2 | 17q23.2 |
| 101 | YWHAH | tyrosine 3-monooxygenase/ tryptophan 5-monooxygenase activation protein, eta polypeptide | BC003047 | 7533 | YWHA1 | NM_003405 | Hs.22675 5 | 22q12.3 |
| 102 | ZDHHC9 | zinc finger, DHHC domain containing 9 | NM_016032 | 51114 | CGI-89, ZNF379 | NM_016032 | Hs.27435 1 | 9 |

In a particular embodiment of a method according to the invention, said method may further comprise determining from a biological sample of the subject at least one additional parameter useful for the diagnosis. Such "parameters useful for the diagnosis" are parameters that cannot be used alone for a diagnosis but that have been described as displaying significantly different values between tolerant grafted subjects and subjects in chronic rejection and may thus also be used to refine and/or confirm the diagnosis according to the above described method according to the invention. They may notably be selected from:
- standard biological parameters specific for said subject grafted organ type,
- phenotypic analyses of peripheral blood mononuclear cells (PBMC), and
- qualitative and/or quantitative analysis of PBMC immune repertoire.

According to the invention, "standard biological parameters specific for said subject grafted organ type" means biological parameters that are usually used by clinicians to monitor the stability of grafted subjects status and to detect graft rejection. These standard biological parameters specific for said subject grafted organ type usually comprise serum or plasma concentrations of particular proteins, which vary depending on the grafted organ type. However, these standard biological parameters specific for said subject grafted organ type are, for each organ type, well known of those skilled in the art.

For instance, standard biological parameters specific for kidney include serum or plasma urea and creatinine concentrations. In a healthy subject, the serum creatinine concentration is usually comprised between 40 to 80 µmol/L for a woman and 60 to 100 µmol/L for a man, and the serum urea concentration between 4 to 7 mmol/L.

For instance, for liver transplantation, standard biological parameters include serum or plasma concentrations of gamma glutamyl transpeptidase (GGT), aspartate aminotransferase (AST), alanine aminotransferase (ALT), lactate dehydrogenase (LDH), and bilirubin (total or conjugated).

These standard biological parameters have the advantage of being easily measurable from a blood sample, but are not sufficient to establish a precise graft tolerant or non-tolerant diagnosis, and are also not enough sensitive to allow an early chronic rejection diagnosis. However, when combined with the determination of an expression profile according to the present invention, the resulting method according to the invention makes it possible to detect graft tolerant subject whose immunosuppressive treatment could be progressively decreased, as well as apparently stable patients (relative to their biological parameters) who are potentially actually on the verge of chronic rejection.

The phenotypic analyses of peripheral blood mononuclear cells (PBMC) may comprise various types of phenotypic analysis. In particular they may comprise:
- measuring the percentage of CD4⁺ CD25⁺ T cells in peripheral blood lymphocytes, which may be performed by any technology known in the art, in particular by flow cytometry using labelled antibodies specific for the CD4 and CD25 molecules. Preferably, the percentage of CD4⁺ CD25⁺ T cells in peripheral blood lymphocytes of a tolerant subject is not statistically different from that of a healthy volunteer, whereas it is significantly lower (p < 0.05) in a non-tolerant grafted subject (23).
- determining the cytokine expression profile of T cells, which may be performed using any technology known in the art, including quantitative PCR and flow cytometry analysis. Preferably, the oligoclonal Vβ families of a non-tolerant grafted subject express increased levels compared to a healthy volunteer of TH1 or TH2 effector molecules, including interleukin 2 (IL-2), interleukin 8 (IL-8), interleukin 10 (IL-10), interleukin 13 (IL-13), transforming growth factor beta (TGF-β), interferon gamma (IFN-γ) and perforin, whereas oligoclonal Vβ families of a tolerant grafted subject do not express increased levels of those effector molecules compared to a healthy volunteer (2).

The analysis of PBMC immune repertoire consists advantageously in the qualitative and quantitative analysis of the T cell repertoire (2), such as the T cell repertoire oligoclonality and the level of TCR transcripts or genes.

The T cell repertoire oligoclonality may be determined by any technology enabling to quantify the alteration of a subject T cell repertoire diversity compared to a control repertoire. Usually, said alteration of a subject T cell repertoire diversity compared to a control repertoire is determined by quantifying the alteration of T cell receptors (TCR) complementary determining region 3 (CDR3) size distributions. In a healthy subject, who can be considered as a controle repertoire, such a TCR CDR3 size distribution displays a Gaussian form, which may be altered in the presence of clonal expansions due to immune response, or when the T cell repertoire diversity is limited and reaches oligoclonality.

The level of TCR expression at the genomic, transcriptionnal or protein level is preferably determined independently for each Vβ family by any technology known in the art. For instance, the level of TCR transcripts of a particular Vβ family may be determined by calculating the ratio between these Vβ transcripts and the transcripts of a control housekeeping gene, such as the HPRT gene. Preferably, in a graft tolerant subject, a significant percentage of Vβ families display an increase in their transcript numbers compared to a normal healthy subject.

An example of methods to analyze T cell repertoire oligoclonality and/or the level of TCR transcripts, as well as scientific background relative to T cell repertoire, are clearly and extensively described in WO 02/084567 (24), which is herein incorporated by reference. Preferably, a graft tolerant subject, as well as a subject in chronic rejection, displays a T cell repertoire with a significantly higher oligoclonality than a normal healthy subject.

Such additional parameters may be used to confirm the diagnosis obtained using the expression profile comprising or consisting of the 8 genes from Table 1. For instance, when the subject is a kidney grafted subject, certain values of the standard biological parameters may confirm a graft non-tolerant diagnosis: if the serum concentration of urea is superior to 7 mmol/L or the serum concentration of creatinine is superior to 80 µmol/L for a female subject or 100 µmol/L for a male subject, then the tested subject is diagnosed as not tolerant to his graft.

In a preferred embodiment of any above described in vitro diagnosis method according to the invention, said subject is a kidney transplanted subject. According to the invention, a "kidney transplanted subject" is a subject that was grafted with a non syngeneic, including allogenic or even xenogenic, kidney. Said kidney transplanted subject may further have been grafted with another organ of the same donor providing the kidney. In particular, said kidney transplanted subject may further have been grafted with the pancreas, and optionally a piece of duodenum, of the kidney donor.

In another preferred embodiment of any above described in vitro diagnosis method according to the invention, said subject is a liver transplanted subject. According to the invention, a "liver transplanted subject" is a subject that was grafted with a non syngeneic, including allogenic or even xenogenic, liver. Said liver transplanted subject may further have been grafted with another organ of the same donor providing the liver.

The invention further concerns a kit for the in vitro diagnosis of a graft tolerant or graft non-tolerant phenotype, comprising at least one reagent for the determination of an expression profile comprising, or consisting of, the 8 genes from Table 1. In some embodiments, the reagent(s) permit for the determination of an expression profile further comprising at least one (and in some cases all) gene(s) from Table 2. By "a reagent for the determination of an expression profile" is meant a reagent which specifically allows for the determination of said expression profile, i.e. a reagent specifically intended for the specific determination of the expression level of the genes comprised in the expression profile. This definition excludes generic reagents useful for the determination of the expression level of any gene, such as taq polymerase or an amplification buffer, although such reagents may also be included in a kit according to the invention.

Such a kit for the in vitro diagnosis of a graft tolerant or graft non-tolerant phenotype may further comprise instructions for determination of the presence or absence of a graft tolerant phenotype.

Such a kit for the in vitro diagnosis of a graft tolerant phenotype may also further comprise at least one reagent for the determining of at least one additional parameter useful for the diagnosis such as the expression profile obtained from the analysis of at least one gene (for instance 1, 2, 3, 4, 5, 6, 7 or more, such as about 10, 15, 20, 25, 30 or even 40, 50, 60, 70, 80 or even advantageously the 102 genes) of Table 3, standard biological parameters specific for said subject grafted organ type, phenotypic analyses of PBMC (notably the percentage of CD4⁺ CD25⁺ T cells in peripheral blood lymphocytes and the cytokine expression profile of T cells), and quantitative and/or qualitative analysis of PBMC immune repertoire (such as the T cell repertoire oligoclonality and the level of TCR transcripts).

In any kit for the in vitro diagnosis of a graft tolerant phenotype according to the invention, the reagent(s) for the determination of an expression profile comprising, or consisting of, the 8 genes from Table 1, and optionally at least one gene from Table 2, preferably include specific amplification primers and/or probes for the specific quantitative amplification of transcripts of genes of Table 1 and optionally of Table 2, and/or a nucleic microarray for the detection of genes of Table 1 and optionally of Table 2. The determination of the expression profile may thus be performed using quantitative PCR and/or a nucleic microarray, preferably an oligonucleotide microarray.

In addition, the instructions for the determination of the presence or absence of a graft tolerant phenotype preferably include at least one reference expression profile. In a preferred embodiment, at least one reference expression profile is a graft tolerant expression profile. Alternatively, at least one reference expression profile may be a graft non-tolerant expression profile. More preferably, the determination of the level of graft tolerance is carried out by comparison with both graft tolerant and graft non-tolerant expression profiles as described above.

The invention is also directed to a nucleic acid microarray comprising or consisting of nucleic acids specific for the 8 genes from Table 1. Said nucleic acid microarray may further comprise at least one nucleic acid specific for at least one gene from Table 2. In particular, it may comprise nucleic acids specific for the 41 genes from Table 2. Said nucleic acid microarray may comprise additional nucleic acids specific for genes other than the 8 genes from Table 1, but preferably consists of a maximum of 500, 400, 300, 200 preferably 100, 90, 80, 70 more preferably 60, 50, 40, even more preferably 30, 25, 20, 15, or 10 distinct nucleic acids, 8 of which are specific for the 8 genes of Table 1. Advantageously, said microarray consists of the 8 genes of Table 1. In a preferred embodiment, said nucleic acid microarray is an oligonucleotide microarray comprising or consisting of oligonucleotide specific for the 8 genes from Table 1.

The invention is also drawn to a method of treatment of a grafted subject, comprising:
(a) determining from a subject biological sample the presence of a graft tolerant or graft non-tolerant phenotype using a method according to the invention, and
(b) adapting the immunosuppressive treatment in function of the result of step (a).

Said adaptation of the immunosuppressive treatment may consist in:
- a reduction or suppression of said immunosuppressive treatment if the subject has been diagnosed as graft tolerant, or
- a modification of said immunosuppressive treatment if the subject has been diagnosed as developing a chronic rejection.

Having generally described this invention, a further understanding of characteristics and advantages of the invention can be obtained by reference to certain specific examples and figures which are provided herein for purposes of illustration only and are not intended to be limiting unless otherwise specified.

### DESCRIPTION OF THE DRAWINGS

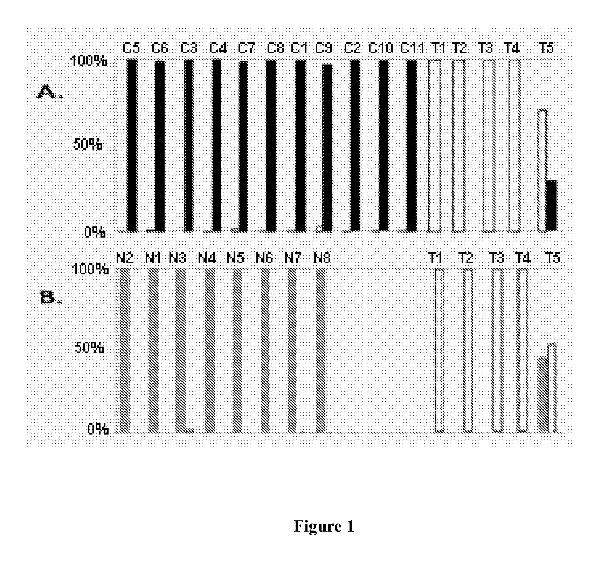

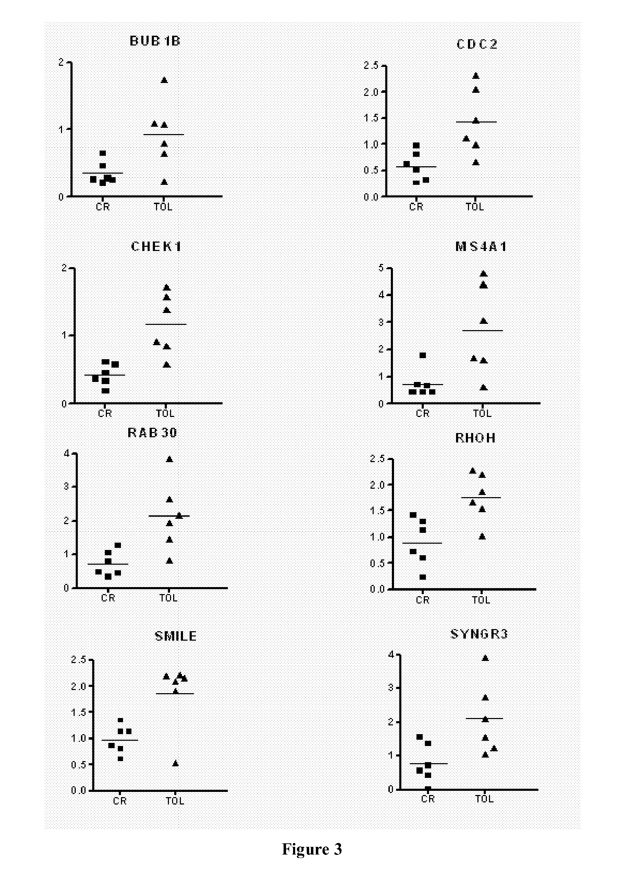

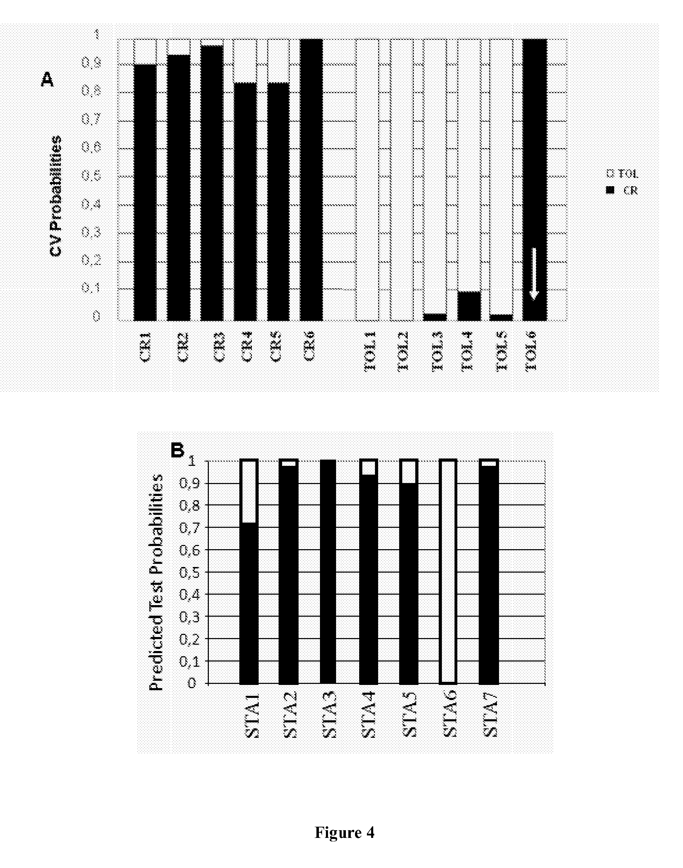

***Figure 1******. Identification and Prediction of "tolerance genes" in patient samples using a subset of 49 known unique genes: 3-Class analysis of samples from tolerating patients (T), patients with chronic rejection (C) and healthy individuals (N) :***
   Each patient sample is shown by a bar and identified in the X-axis. The Y axis indicates the predicted probability (0-100%) that the sample belongs to tolerating patients (white bar), patients with chronic rejection (black bar), or healthy volunteer (grey bar).
      ***1A). Tolerance prediction by 2-Class comparison of tolerating and rfejecting patients.*** Figure 1A displays a cross-validated comparison of a training set of 5 tolerating patients (T1-5) and 11 patients with chronic rejection (C5-C11). All samples have a 100% fit to phenotype across the 49 selected gene set, except patient T5, who has ~80% fit-to-class scores.
      ***1B). Tolerance prediction by 2-Class comparison of tolerating patienrts and healthy individuals.*** A cross-validated comparison of a training set of blood samples from 5 tolerating patients (T1-5) and 8 healthy volunteers (N1-8), again shows sample T5 as the only weak classifier.

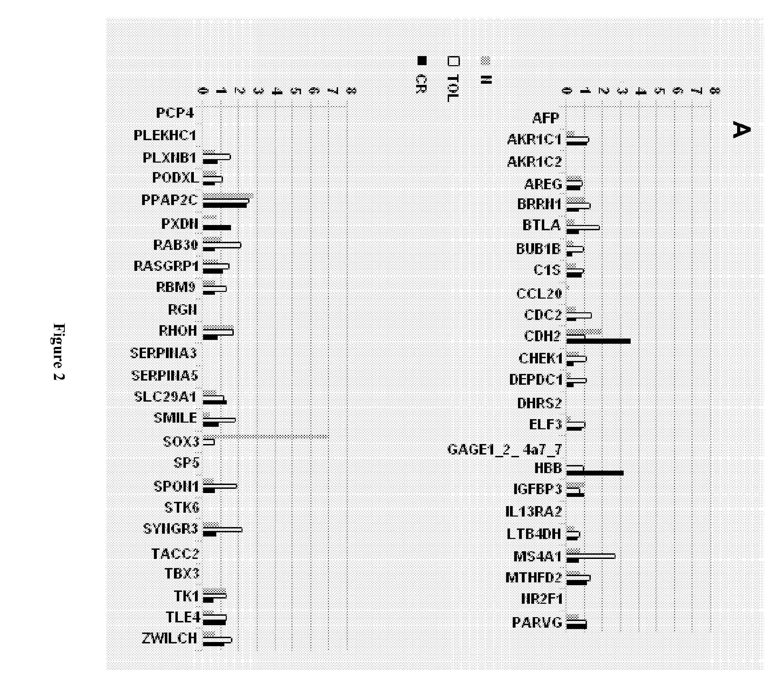

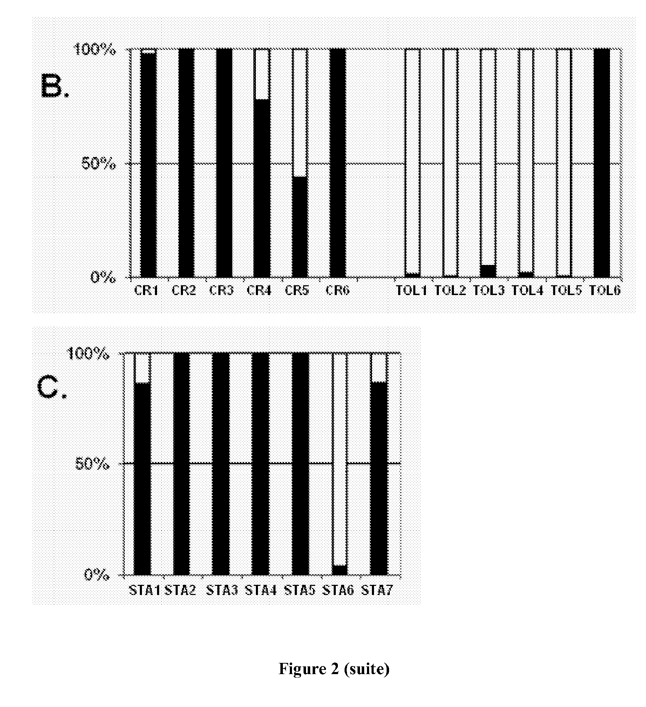

***Figure 2******. Testing the predictive power of the tolerance footprint using RT-PCR in independent patients with chronic rejection (CR) and tolerating (TOL) patients:***
   ***2A) Mean expression of 49 genes in whole blood total RNA relative to GAPDH expression levels.*** Real time quantitative PCR analysis using Taqman RT-PCR assays was done on the 49 selected gene set, across 3 patient groups including the original Normal controls (N; n=6, grey bars), an independent group of CR patients (CR; n=6, black bars), as well as a second independent group of TOL-Test patients (TOL; n=6, white bars). Data on the 49 genes is shown in figure 2A. The expression level of each gene was calculated according to the 2^{-ΔΔCt} method following normalization to a housekeeping gene and using a pool of patients with stable graft function as the reference sample. Triplicate measurements were averaged and expression normalized to levels of GAPDH. The mean fold expression normalized by GAPDH and relative to a reference sample is reported for each group..
   ***2B) Reduced tolerance footprint predictive of a potential tolerant state in TOL and CR transplant patients using RT-PCR.*** A two-class analysis (by PAM) for chronic rejection (black bars) or tolerance phenotype (white bars) was done for 33 out of the 49 gene expression measurements obtained by quantitative PCR. 16 genes were not included in the 2-class PAM analysis because data were not obtained by quantitative PCR for at least 75% of the samples analysed in these genes. As shown most patients fit to class well, while TOL6 is predicted as chronic rejection. Interestingly, whereas this last patient fulfilled the operationally tolerant state criteria at the time of harvesting (two years prior to the PCR study), he has since then started to decline graft function.
   ***2C) Three-class Prediction for stable transplant patients on Immunosuppression.*** Class prediction for tolerance (white bars), when applied across 7 stable (STA) patients (STA1 - STA7), using the 33 PCR gene expression measurements, predicts patient STA6 as being tolerant.
**Figure *3******. Significant gene expression of 8 genes.*** A t-test, an anova and a Kruskal-Wallis tests were performed on the 33 genes. According to these tests 8 genes were found to be highly significative between TOL and CR patients (p<0.05).
***Figure 4******. Minimal tolerance footprint predictive of a potential tolerant state in TOL vs CR transplant patients using RT-PCR.***
   A) ***Two-class PAM analysis of CR and TOL patients.*** The 8 genes retained on their significance (p<0.05) (Figure 3) were used in a cross-validated PAM two class analysis and blindly correctly classified new tolerant (white bars) and new CR (black bars), with a single misclassification (TOL6 as CR) (Figure 4A). As previously mentioned, TOL6 fulfilled the full clinical description of operationally tolerance, 2 years prior to and at the time of harvesting but 6 months after testing decline in renal function was observed. If this patient is eliminated from the statistical analysis, a 93,89% sensitivity (P_{TOL}=96,58%; P_{CR}=91,66%) and 100% specificity are obtained.
   ***B) Three-class Prediction for stable transplant patients on Immunosuppression.*** Class prediction for tolerance (white bars), when applied across the 7 stable (STA) patients (STA1 - STA7), using the 8 PCR gene expression measurements, predicts, as previously obtained, patient STA6 as being tolerant (Figure 4B).

### EXAMPLE 1

### Analysis of drug-free operational immune tolerance in human kidney graft recipients by gene expression profiling

### Patients, Materials and Methods

### Patient Selection

Peripheral blood samples were collected from 43 various adult renal transplant patients groups (tolerant patients, patients with chronic rejection, and patients with stable graft function under immunosuppression; Table 4) and 14 normal adult controls. The protocol was approved by an Ethical Committee and all patients signed a written informed consent before inclusion. Samples were separated into Training-group (analysed by microarray) and Test-group (analysed by real-time quantitative PCR) cohorts containing patient with different clinical phenotypes. Apart from tolerant patients for whom biopsy was refused by the Hospital Ethical Committee, all other patients had biopsy-confirmed clinical phenotypes.

**Table 4. Demographic summary of patient groups (Median and range).**

| | **Training Groups** | | | **Test Groups** | | | |
|---|---|---|---|---|---|---|---|
| | **TOL** | **CR** | **Normal** | **TOL-Test** | **CR-Test** | **Stable** | **Test-N** |
| Number | 5 | 11 | 8 | 6 | 6 | 7 | 14 |
| | 67 | 56 | 23 | 38.5 | 57.5 | 54 | 46 |
| Age (years) | 58-73 | 28-75 | 11-27 | 25-74 | 52-59 | 31-72 | 30-66 |
| % Male | 80% | 63.60% | 37.5% | 66% | 66% | 42.8% | 0% |
| Time post- | 178 | 59 | NA | 137 | 98 | 65 | NA |
| Transplant (mo) | 108-360 | 20-158 | | 56-372 | 42-158 | 23-236 | |
| Serum | 122 | 244 | NA | 109 | 280.5 | 104 | NA |
| Creatinine (µM/1) | 82-139 | 127-492 | | 82-139 | 127-492 | 68-147 | |
| Proteinuria | 0.83 | 1.93 | NA | 0.225 | 2.71 | 0.1 | NA |
| per day (g/24h) | 0-1.28 | 0.34-11.75 | | 0.0-0.93 | 0.56-11.75 | 0-0.25 | |
| Prior AR | 20% | 36% | NA | 33% | 16.6% | 14.3% | NA |
| Prior CA | 20% | 0% | NA | 17% | 0% | 0% | NA |
| Prior CMV | 0% | 27% | NA | 0% | 16.6% | 28.6% | NA |
| HLA | 3.2 | 3 | NA | 3 | 2 | 4 | NA |
| incompatibi lies | 03-4 | 01-5 | | 0-4 | 01-5 | 0-4 | |

| | | | | | | | |
|---|---|---|---|---|---|---|---|
| TOL - Tolerance; CA - Cancer, CR - Chronic Rejection; STA - Stable function; NA - Not Applicable. | | | | | | | |

To generate informative biomarkers by microarray for operational tolerance, **Training-group** samples (n=24) were chosen from 3 clinical phenotypes:
***1) Immunosuppressive drug-free, operationally tolerant (T, n=5):*** patients with long-term stable graft function without any immunosuppression for at least 2 years (mean duration drug-free= 8.8+/-4.9 years). Stable graft function was defined as stable Cockcroft calculated creatinine clearance > 60 mls/min/1.73m2 with absent or low grade proteinuria (<1.5g/day) (2). The clinical and biological characteristics of these patients have been described in detail previously (25) and the most relevant demographic and clinical data of the entire population studied are summarized in Table 4.
***2) Chronic rejection (C, n=11):*** All patients had a progressive degradation of their renal function (creatinine clearance < 60 mls/min/1.73m2 and/or proteinuria >1.5g/day) and histological signs of vascular chronic rejection defined as endarteritis and allograft glomerulopathy with basal membrane duplication. Four out of 11 patients were on dialysis due to irreversible loss of graft function, and patients from this group had completely stopped their immunosuppressive treatment for 1.5+/-0.5 years. Demographic and clinical data of these patients are shown in Table 4.
***3) Age-matched healthy volunteers (N=8)*** were included as controls. They all had a normal blood formula and no infectious or other concomitant pathology for at least 6 months prior to the study (Table 4).

To allow for validation of the discovered biomarkers for operational tolerance, an independent, blinded Test-group of samples (N=53) from 4 different phenotypes were examined by expression profiling using real-time PCR. The nomenclature and definitions of these different test-group cohorts are as follows:
***1) Immunosuppressive drug-free operationally tolerant test-group (TOL; N=6):*** all new patients shared the same clinical and pathological criteria as described above (Table 4). All stopped their immunosuppression for non-adherence reasons.
***2) Chronic rejection test-group (CR, N=6)***. all new patients shared the same clinical and pathological criteria as described above (Table 4).
***3) Long-term stable test-group (STA, N=7)*:** patients with stable kidney graft function at > 5 years post-transplantation while under mycophenolate mofetil or azathioprine, and maintenance steroids with or without an associated calcineurin inhibitor.
***4) Age-matched healthy volunteers (N, N=6).*** They all had a normal blood formulae and no infectious or other concomitant pathology for at least 6 months prior to the study.

Demographic and clinical data for all these patients are shown in Table 4.

### Microarray Experiments

Ten milliliter of peripheral blood was collected in EDTA tubes. Peripheral Blood Mononuclear Cells (PBMC) were separated on a Ficoll layer (Eurobio, Les Ulis, France) and frozen in Trizol® reagent (Invitrogen, Life technologies, California). To obviate gene expression bias based on sample collection methods, whole blood from some patients was directly tested. RNA was extracted according to the manufaturer protocol. cDNA microarrays, containing ~32,000 cDNA clones (12,400 known unique genes) were processed using 2 µg RNA in each channel against a "common reference" RNA pool. Significance Analysis of Microarray (SAM) 2-class was used to determine significant differential gene expression between each patient group. The Cluster program (26) was used to identify gene patterns and clusters. Enrichment of functional gene classes was identified using Expression Analysis Systematic Explorer (EASE); *http:*//*appsl.niaid.nih.gov*/*david*/*)* and by hypergeometric enrichment analysis. Predictive analysis of Microarray or PAM class prediction (27) was used to determine the "expression phenotypes" of the unidentified, independent test group samples.

### Quantitative Real-time PCR gene expression validation

PCR primers and probes were designed to the 49 genes (tolerance "footprint" from the microarray screen) and GAPDH, the normalizing housekeeping genes. Amplified and total RNA (100 ng) was subjected to real-time RT-PCR analysis. Quantitative PCR was performed in triplicate in an Applied Biosystems GenAmp 7700 sequence detection system (Applied Biosystems, Foster City, CA). A full list of these genes and their accession numbers are displayed in Table 1 and Table 2.

### Statistics

Wilcoxon rank sum test (p<0.05 used for significance), logistic regression and Pearson's correlation test (expressed as R2) were run on the clinical data.

### Results

### Biomarker Discovery and Biomarker Validation for a tolerance footprint

Microarray analysis using a minimal gene-set of 59 transcripts representing 49 clinically relevant unique genes was performed on 24 training-group peripheral blood samples (5 T, 11 C and 8N).

Two-class prediction tests using the PAM (version 2) class prediction tool (27) were applied between tolerating and rejecting patients (Figure 1A). Except a weaker classification for patient T5, the remainder of the samples have >80% match scores by gene expression to their phenotype. (Figure 1A).

PAM 2-class prediction was next applied across blood samples from tolerating patients and healthy individuals, using the minimal gene-set of 49 clinically relevant unique genes in order to assess if the gene expression profile could also discriminate between both groups of patients (Figure 1B). This makes it possible to ascertain that the 49 gene set was the hallmark of operationally tolerant and was not due to the absence of immunosuppressive drug in the tolerating patients. The expression of these genes classifies most tolerant patients accurately. None Healthy individuals score as tolerant in this analysis, while one operationally tolerant patients do (fit-to-phenotype scores of >90%; T5 scored ~50%; Figure 1B).

Collectively, these data indicate that the discriminative power of the gene expression profile (Table 5) is robust enough to classify tolerating patients correctly in this experiment with a specificity of 100% and sensitivity of 90%.

**Table 5. Expression of minimal gene set (tolerance footprint) that differentiates tolerance, CR and normal blood.**

| **Symbol** | **TOL vs. N** | **TOL vs. CR** |
|---|---|---|
| AFP | 5.09 | 4.40 |
| AKR1C1° | 4.02 | 4.45 |
| AKR1C2° | 4.08 | 4.35 |
| AREG° | 7.35 | 1.62 |
| BRRN1 ° | 5.27 | 2.88 |
| BTLA° | 0.22 | 0.76 |
| BUB1B ° | 3.87 | 2.22 |
| C1S° | 9.08 | 3.66 |
| CCL20° | 10.79 | 6.17 |
| CDC2° | 6.06 | 2.96 |
| CDH2° | 8.54 | 5.61 |
| CHEK1° | 6.02 | 2.97 |
| D2S448 | 5.24 | 3.96 |
| DEPDC1° | 4.96 | 2.54 |
| DHRS2° | 8.15 | 7.47 |
| ELF3 ° | 6.34 | 2.92 |
| FLJ10036 | 3.50 | 2.34 |
| GAGE2 | 5.04 | 4.20 |
| HBB° | 0.14 | 0.43 |
| IGFBP3 ° | 4.51 | 2.63 |
| IL13RA2 | 5.48 | 3.10 |
| LTB4DH ° | 6.57 | 4.08 |
| MS4A1 ° | 0.18 | 0.88 |
| MTHFD2° | 4.54 | 2.23 |
| NR2F1 | 4.97 | 3.92 |
| PARVG° | 4.78 | 5.17 |
| PCP4 | 8.12 | 7.15 |
| PLEKHC1 | 6.55 | 2.53 |
| PLXNB1° | 4.61 | 2.16 |
| PODXL° | 5.88 | 2.68 |
| PPAP2C° | 9.55 | 4.95 |
| RAB30° | 0.16 | 0.56 |
| RASGRP1° | 0.17 | 0.19 |
| RBM9° | 9.07 | 6.70 |
| RGN | 7.81 | 4.03 |
| RHOH° | 0.13 | 0.27 |
| SERPINA3 | 5.24 | 3.51 |
| SERPINA5 | 6.24 | 6.14 |
| SLC29A1° | 3.47 | 2.02 |
| SMILE° | 4.37 | 2.53 |
| SOX3° | 4.55 | 3.51 |
| SP5 | 7.37 | 3.81 |
| SPON1° | 5.70 | 4.68 |
| STK6 | 4.80 | 4.24 |
| SYNGR3° | 3.35 | 2.19 |
| TACC2 | 3.78 | 2.93 |
| TBX3 | 5.07 | 3.34 |
| TK1° | 5.02 | 5.29 |
| TLE4 | 0.15 | 0.17 |

| | | |
|---|---|---|
| ° are the 33 genes found the most expressed by quantitative PCR. | | |

### "Minimal tolerance footprint" predictive of a potential tolerant state in stable transplant patients using RT-PCR

Quantitative RT-PCR on the 49 genes from the tolerance microarray dataset and GAPDH were performed in triplicate on RNA extracted from the PBMC of 6 independent TOL-Test patients (TOLI-TOL6) and 6 independent CR-Test patients (CR1-CR6), none of whom were included in microarray analysis as well as from the PBMC of 6 healthy individuals (Figure 2A). Seven stable transplant patients (STA1-STA7) were also analysed by QPCR using this set of genes. To exclude biases due to the amplification of the RNA for the microarray analysis, these PCR experiments were performed on non-amplified RNA extracted from the PBMC of the patients.

A composite model of the 33 most abundant PCR gene expression measurements out of the 49 genes analysed were used in a cross-validated PAM two class analysis and blindly correctly classified the tolerating (white bars) and rejecting patients (black bars), with a single misclassification (TOL6 as CR) (Figure 2B). Interestingly, although TOL6 fulfilled the full clinical description of operationally tolerance, 2 years prior to and at the time of harvesting, 6 months after testing, a decline in his renal function was observed (creatinemia: 165 µm/l, proteinuria: 1g/day), with demonstration of anti-donor class II (anti-HLA DR4) antibodies. This clinical picture suggests that the operational tolerance gene expression signature is likely a meta-stable, rather than a permanent state.

Composite PCR expression of the 33 genes was next used to classify 7 stable post-transplant patients as TOL or CR. Consistent with the microarray-based classification, a single stable patient (STA6) was predicted to share the TOL phenotype with a classification score >99% (0.996) (Figure 2C). Thus, the peripheral blood signature of operational tolerance in renal transplant recipients is robust and can be identified by PCR based gene-expression profiling across a modest number of genes in peripheral blood.

### Discussion

Kidney transplantation remains the major treatment for end-stage renal diseases but is often complicated either by acute or chronic rejection or by side effects of the long-term immunosuppression. The molecular basis of these processes have been analyzed by gene expression profiling in various studies focusing on acute or chronic rejection and response to treatment (28), demonstrating the unique potential of this approach to decipher complex pathological processes in human disease. In contrast, the gene expression and corresponding molecular pathways have never been investigated in operational tolerance in human, which can be considered as a model for drug-free kidney transplantation. Recently, the feasibility and value of microarray analysis of operational tolerance has been demonstrated by Martínez-Llordella and colleagues in liver transplantation (29).

A major implication of the description of a specific gene expression profile in operationally tolerant patients is its potential use to identify patients who may benefit from progressive minimization of immunosuppression without major risk for rejection. Indeed, operational tolerance in kidney transplantation may be masked in long-term patients under immunosuppression and the identification of a specific biological signature of tolerance could open new perspectives for rational rather than empiric minimizing of immunosuppressive drugs in well-selected patients.

In the present study, we combined the availability of a unique cohort of operationally tolerant kidney graft recipients with the power of high throughput gene expression profiling to study the blood molecular pattern associated with operational tolerance. "Operational tolerance" is defined by a well functioning graft in an immunocompetent host in the absence of immunosuppression (25). We previously showed that the operationally tolerant patients studied are healthy, free of infection and malignancy and do not display clinical evidence of immunoincompetency (25), in so far as the ability to mount a normal or sub-normal response to flu vaccination is observed. Nevertheless, the fact that operational tolerance definition refers to a clinical status, precludes a possible response of the recipient against his donor and nothing proves that operational tolerance will be indefinite.

Our study provides, for the first time, a novel and non invasive transcriptional assay for monitoring the recipient's level of immune adaptation to the donor organ. In particular, this study allowed to validate a *specific biomarker footprint of tolerance* where peripheral tolerance is predicted with >90% fit-to-class scores, in an independent set of immunosuppressive drug-free operationally tolerant patients, as well as a sub-set of patients with stable graft function under immunosuppressive therapy. In this study, interestingly, the patient TOL6 was predicted as chronic rejection in the cross-validation test. However, whereas this patient fulfilled the operationally tolerant state at the time of harvesting and since two years, he started to decline his graft function 6 months after testing (creatinemia: 165 µm/l, proteinuria: 1 g/day) and appearance of anti-donor class II (antiDR4) antibodies. This patient refused biopsy precluding an accurate diagnosis of chronic rejection. However, his transcriptional profile and class prediction scoring distinguish him from other TOL patients even prior to eventual decline in graft dysfunction. This observation suggests that an absence of the tolerance signature could possibly be used in a prognostic way. Further, the loss of the peripheral signature for tolerance correlates clinically to a change in clinical phenotype from operational tolerance to rejection. For the first time, we may be able to define the patients who could be eligible for a progressive decrease of their immunosuppressive medications and more importantly, identify the patients who need to stay on their current immunosuppression dose.

Several strategies were used to ascertain the robustness and reproducibility of the obtained gene expression profile in operationally tolerant patients. Firstly, all quantitative PCR analyses were done in triplicate. Secondly, although still relatively small due to the extreme scarcity of spontaneous tolerance in kidney transplantation, the 11 patients (one of the largest group so far reported) with operational tolerance and the 17 patients with biopsy documented chronic rejection were carefully matched for age. Moreover, both chronic rejection and healthy volunteers were used as comparators in order to ascertain that the obtained profile was specific for operational tolerance and not just for absence of immunosuppression or good renal function. Thirdly, the gene expression obtained by microarray was confirmed on non-amplified RNA samples by an independent technique using quantitative PCR. Finally, the obtained gene profile was validated on patient cohort independent from the one used to identify the set of genes. According to these different points, we identified a minimal list of 8 genes able to discriminate operational tolerance from chronic rejection.

Microarray profiles have already been shown to have a high predictive diagnostic or prognostic value in other pathological conditions such as breast cancer (30). Here, we demonstrated that the obtained gene expression profile classified correctly more than 95% of the samples in a cross-validation experiment. More importantly, the expression algorithm still yielded a positive predictive value for operational tolerance of >80% in a complete independent cohort of both operationally tolerant and chronic rejection patients.

Defining a gene pattern associated with operational tolerance in the human opens new perspectives which, in combination with previously described blood derived biomarkers, such as TCR profiles (2) and lymphocyte phenotyping (23) in the same cohorts of patients, may help to identify patients under immunosuppression with low immunological risk of rejection. The fact that the described gene expression profile has been obtained from PBMC is of major importance in a clinical perspective and can thus be easily used and also transposed and validated in other settings. Ongoing studies are therefore using these non-invasive immunomonitoring methods to assess the frequency of potentially tolerant patients in large cohorts of kidney graft recipients with stable renal function under standard immunosuppression.

### EXAMPLE 2

### Further reduction of the peripheral blood "footprint" of operational tolerance to a set of 8 genes

### Patients, Materials and Methods

### Patients

Test-group samples: 1) Immunosuppressive drug-free operationally tolerant test-group (TOL; N=6): patients with long-term stable graft function without any immunosuppression for at least 2 years (mean duration drug-free= 8.8+/-4.9 years). 2) Chronic rejection test-group (CR, N=6): All patients had a progressive degradation of their renal function (creatinine clearance < 60 mls/min/1.73m² and/or proteinuria >1.5g/day) and histological signs of vascular chronic rejection defined as endarteritis and allograft glomerulopathy with basal membrane duplication. 3) Patients with stable graft function under immunosuppression (STA, N=7) (Table 4).

### Quantitative Real-time PCR gene expression validation

PCR primers were designed to the 49 genes (tolerance "footprint" from the microarray screen) and GAPDH,the normalizing housekeeping gene. Non-amplified RNA was treated with DNase (Roche, Indianapolis, USA). Quantitative PCR was performed in triplicate in an Applied Biosystems GenAmp 7700 sequence detection system (Applied Biosystems, Foster City, CA).

### Statistics

Anova, t-test and Kruskal Wallis test (p<0.05 used for significance) were used to select RT-PCR significant genes.

### Results

### Minimal tolerance footprint able to differentiate tolerating and rejecting patients

Quantitative RT-PCR for the 49 genes from the tolerance microarray dataset (see Example 1) and GAPDH were performed in triplicate on RNA extracted from the PBMC of 6 independent TOL-Test patients (TOL1 to TOL6) and 6 independent CR-Test patients (CR1 to CR6), none of whom were included in microarray analysis. Eight of these genes were found statistically significant for the tolerance group when compared to the CR group (p< 0.005). These genes are BUB1B, CDC2, CHEK1, MS4A1, RAB30, RHOH, SMILE, SYNGR3 (Figure 3). These results were obtained by applying a t-test, an anova and a Kruskal-Wallis tests on the 33 genes found the most accumulated by quantitative PCR.

A two-class PAM predictive test was applied on these independent tolerating and rejecting patients and showed a very good classification of both groups of patients on the basis of the analysis of only 8 genes (Figure 4A). Interestingly, as previously observed (Figure 2C), although TOL6 fulfilled the full clinical description of operationally tolerance, 2 years prior to and at the time of harvesting, 6 months after testing decline in renal function was observed (creatinemia: 165 µm/l, proteinuria: 1g/day), with demonstration of anti-donor class II (anti-HLA DR4) antibodies. If this patient is eliminated from the statistical analysis, a 93,89% sensitivity (P_{TOL}=96,58%; P_{CR}=91,66%) and 100% specificity are obtained.

### Minimal tolerance footprint predictive of a potential tolerant state in stable transplant patients using RT-PCR

A three-class PAM predictive test was then applied using the patients twithon these independent tolerating and rejecting patients and showed a very good classification of both groups of patients on the basis of the analysis of only 8 genes (Figure 4A). Interestingly, as previously observed (Figure 2C), although TOL6 fulfilled the full clinical description of operationally tolerance, 2 years prior to and at the time of harvesting, 6 months after testing decline in renal function was observed (creatinemia: 165 µm/l, proteinuria: 1g/day), with demonstration of anti-donor class II (anti-HLA DR4) antibodies. If this patient is eliminated from the statistical analysis, a 93,89% sensitivity (P_{TOL}=96,58%; P_{CR}=91,66%) and 100% specificity are obtained.

PCR expression of the 8 genes was next used to classify the 7 stable post-transplant patients (STA to STA7) as TOL or CR. Consistent with the previous observation (Figure 2C), a single stable patient (STA6) was predicted to share the TOL phenotype with a classification score >99% (0.996) (Figure 4B). Thus, the peripheral blood signature of operational tolerance in renal transplant recipients is robust and can be identified by PCR based gene-expression profiling across a modest number of genes in peripheral blood

Together these data suggest that we have identified a modest number of 8 genes discriminating operational tolerance which can be monitored using real-time RT-PCR.

### BIBLIOGRAPHY

1. Dantal J, Hourmant M, Cantarovich D, Giral M, Blancho G, Dreno B, Soulillou JP. 1998. Effect of long-term immunosuppression in kidney-graft recipients on cancer incidence: randomised comparison of two cyclosporin regimens. Lancet. 351:623-8
2. Brouard S, Dupont A, Giral M, Louis S, Lair D, Braudeau C, Degauque N, Moizant F, Pallier A, Ruiz C, Guillet M, Laplaud D, Soulillou JP. 2005. Operationally tolerant and minimally immunosuppressed kidney recipients display strongly altered blood T-cell clonal regulation. Am. J. Transplant. 5(2):330-340
3. Nankivell BJ, Borrows RJ, Fung CL, O'Connell PJ, Allen RD, Chapman JR. 2003. The natural history of chronic allograft nephropathy. N. Engl. J. Med. 349 (24):2326-33
4. US 5,143,854;
5. US 5,288,644;
6. US 5,324,633;
7. US 5,432,049;
8. US 5,470,710;
9. US 5,492,806;
10. US 5,503,980;
11. US 5,510,270;
12. US 5,525,464;
13. US 5,547,839;
14. US 5,580,732;
15. US 5,661,028;
16. US 5,800,992;
17. WO 95/21265;
18. WO 96/31622;
19. WO 97/10365;
20. WO 97/27317;
21. EP 373 203;
22. EP 785 280;
23. Louis S, Braudeau C, Giral M, Dupont A, Moizant F, Robillard N, Moreau A, Soulillou JP, Brouard S. 2006. Contrasting CD25hiCD4+T cells/FOXP3 patterns in chronic rejection and operational drug-free tolerance. 81(3):398-407.
24. WO 02/084567;
25. Roussey-Kesler G, Giral M, Moreau A, Subra JF, Legendre C, Noel C, Pillebout E, Brouard S, Soulillou JP. 2006. Clinical operational tolerance after kidney transplantation. Am J Transplant. 6(4):736-46.
26. Eisen, M. B., Spellman, P. T., Brown, P. O. & Botstein, D. 1998 Cluster analysis and display of genome-wide expression patterns. Proc Natl Acad Sci 95 (25): 14863-8.
27. Tibshirani, R., Hastie, T., Narasimhan, B. & Chu, G. 2002. Diagnosis of multiple cancer types by shrunken centroids of gene expression. Proc Natl Acad Sci U S A 99(10): 6567-72.
28. Schmid H., Henger A. & Kretzler M. 2006. Molecular approaches to chronic kidney disease. Curr Opin Nephrol Hypertens. 15:123-129
29. Martinez-Llordella M, Puig-Pey I, Orlando G, Ramoni M, Tisone G, Rimola A, Lerut J, Latinne D, Margarit C, Bilbao I, Brouard S, Hernandez-Fuentes M, Soulillou JP, Sanchez-Fueyo A. 2007. Multiparameter immune profiling of operational tolerance in liver transplantation. Am J Transplant. 7(2):309-19
30. Glas, A. M., Floore, A., Delahaye, L. J., Witteveen, A. T., Pover, R. C., Bakx, N., Lahti-Domenici, J. S., Bruinsma, T. J., Warmoes, M. O., Bernards, R., Wessels, L. F. & Van't Veer, L. J. 2006. Converting a breast cancer microarray signature into a high-throughput diagnostic test. BMC Genomics 7: 278.

## Claims

1. Method for the in vitro diagnosis of a graft tolerant or non-tolerant phenotype, comprising:
(a) determining from a grafted subject biological sample an expression profile comprising the 8 genes from Table 1,
(b) comparing the obtained expression profile with at least one reference expression profile, and
(c) determining the graft tolerant or graft non-tolerant phenotype from said comparison.

2. The method of claim 1, wherein said expression profile further comprises at least one gene from Table 2.

3. The method of claim 2, wherein said expression profile further comprises all the 41 genes from table 2.

4. The method of any of claims 1-3, wherein the obtained expression profile is compared to at least one reference expression tolerant and/or not tolerant profile in step (b).

5. The method of any of claims 1-4, further comprising, if said subject is diagnosed as a graft non-tolerant subject, diagnosing from the expression profile if said subject is developing chronic rejection.

6. The method of any of claims 1-5, wherein the expression profile is determined by measuring the amount of nucleic acid transcripts of said gene(s).

7. The method of claim 6, wherein the expression profile is determined using quantitative PCR or an oligonucleotide microarray comprising 8 oligonucleotides specific for the 8 genes from Table 1.

8. The method of any of claims 1-5, wherein the expression profile is determined using a genomic microarray or a proteic microarray.

9. The method according to any of claims 1-8, wherein said biological sample is a blood sample.

10. The method according to any of claims 1-9, wherein said subject is a kidney transplanted subject.

11. The method according to any of claims 1-10, further comprising determining at least one additional parameter selected from standard biological parameters specific for said subject grafted organ type, phenotypic analyses of peripheral blood mononuclear cells (PBMC), and qualitative and/or quantitative analysis of PBMC immune repertoire.

12. The method according to any of claims 10 or 11, further comprising between steps (b) and (c) the steps of:
(b1) obtaining from a grafted subject biological sample an expression profile comprising at least one gene from Table 3, and
(b2) comparing the obtained expression profile with at least one reference expression profile, and
wherein in step (c), the graft tolerant or graft non-tolerant phenotype is determined from the comparison of both step (b1) and step (b2).

13. A kit for the in vitro diagnosis of a graft tolerant phenotype, comprising at least one reagent for the determination of an expression profile comprising the 8 genes from Table 1 and optionally at least one gene from Table 2.

14. The kit of any of claims 13, further comprising at least one reagent for determining at least one additional parameter selected from standard biological parameters specific for said subject grafted organ type, phenotypic analyses of peripheral blood mononuclear cells (PBMC), and qualitative and/or quantitative analysis of PBMC immune repertoire.

15. The kit according to any of claims 13 or 14, further comprising at least one reagent for the determination of an expression profile comprising at least one gene from Table 3.

16. A nucleic acid microarray comprising nucleic acids specific for the 8 genes from Table 1, and optionally at least one nucleic acid for at least one gene from Table 2.

17. The nucleic acid microarray according to claim 16 which is an oligonucleotide microarray.
